(19) Europäisches Patentamt European Patent Office Office européen des brevets

(11) **EP 3 899 541 B1**

(12) **EUROPEAN PATENT SPECIFICATION**

(45) Date of publication and mention of the grant of the patent:
**14.08.2024 Bulletin 2024/33**

(51) International Patent Classification (IPC):
***G01N 33/543*** (2006.01) ***G01N 33/574*** (2006.01)

(52) Cooperative Patent Classification (CPC):
**G01N 33/57423; G01N 33/543;** G01N 2800/52

(21) Application number: **19832674.6**

(22) Date of filing: **19.12.2019**

(86) International application number:
**PCT/EP2019/086472**

(87) International publication number:
**WO 2020/127840 (25.06.2020 Gazette 2020/26)**

(54) **LUNG CANCER PROTEIN EPITOMIC BIOMARKERS**

EPITOMISCHE BIOMARKER FÜR LUNGENKREBSPROTEINE

BIOMARQUEURS ÉPITOMIQUES DE PROTÉINES DU CANCER DU POUMON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2018 EP 18215688**

(43) Date of publication of application:
**27.10.2021 Bulletin 2021/43**

(73) Proprietor: **Biosystems Immunolab Zrt.
4025 Debrecen (HU)**

(72) Inventors:
• **TAKACS, Laszlo Kristof
1085 Budapest (HU)**
• **LAZAR, Jozsef
4440 Tiszavasvari (HU)**
• **KURUCZ, Laszlo Istvan
1034 Budapest (HU)**
• **ANTAL SZALMAS, Peter
4032 Debrecen (HU)**

(74) Representative: **Bourgouin, André et al
Grosset-Fournier & Demachy
54, rue Saint Lazare
75009 Paris (FR)**

(56) References cited:
**EP-A1- 2 772 759 WO-A1-2008/114917
WO-A1-2011/119484 WO-A2-2011/015602
WO-A2-2013/040142 US-A1- 2012 101 002**

• **JIN YANXIA ET AL: "Identification a novel clinical biomarker in early diagnosis of human non-small cell lung cancer", GLYCOCONJUGATE JOURNAL, CHAPMAN & HALL, BOSTON, vol. 36, no. 1, 3 January 2019 (2019-01-03), pages 57 - 68, XP036691425, ISSN: 0282-0080, [retrieved on 20190103], DOI: 10.1007/S10719-018-09853-Z**

Remarks:
The complete document including Reference Table(s) and the Sequence Listing(s) can be downloaded from the EPO website

**Description**

**Domain of the invention**

The present invention relates to compositions and methods for detecting, managing or monitoring lung cancer via plasma protein epitome profiling. The invention is particularly suited for detecting, managing or monitoring lung cancer in human subjects.

**Background of the invention**

Lung cancer is the most common cause of death from cancer and each year 1.4 million new cases are diagnosed worldwide. More than two-thirds of lung cancers are diagnosed at a late stage, when clinical symptoms appear. The overall survival rate after diagnosis ranges from 16 % in the USA to 1.1 % in some regions of Asia and is currently very low due to the late diagnosis of the disease. Although, survival of other cancers has been improving, in the case of lung cancer there is no apparent improvement since the late sixties. Importantly, as smoking habit is still spreading in Asia, lung cancer death rate increases in this region of the world with alarming rates.

Early detection of asymptomatic lung cancer significantly improves survival because likelihood of curative surgery or life prolonging therapy (beyond 5 years) is high at early stages where removal of LC tissue is an option (state I - IIIa) and specially at stage I-II where removal alone or combined with adjuvant chemotherapy may result in complete cure. Current diagnostics methods are based on imaging techniques and invasive procedures such as bronchoscopy or biopsy. The few known plasma biomarkers for lung cancer, such as carcinoembryonic antigen (CEA), cytokeratin-19 (CYFRA), squamous cell carcinoma antigen (SCC) and neuron-specific enolase (NSE) lack sufficient sensitivity and specificity to be used assisting early diagnosis Ongoing efforts using high throughput discovery technologies are still struggling to provide reliable and easily accessible lung cancer biomarkers to enter the clinic.

Today effective screening for the detection of early stage asymptomatic lung cancer is done via computer tomography, especially repeated low dose spiral computer tomography (LDCT). These protocols follow international recommendations and are based on large scale studies in the US and Europe. The sensitive imaging technology does detect 3-4 mm diameter nodules in the lung, however >90% of these are not cancerous, moreover biopsy of small nodules <6-10 mm diameter is not easy; it requires specific instrumentation and skills. Another negative aspect of repeated LDCT is that the cumulated irradiation dose reaches the limit of carcinogenicity. In order to increase the specificity of the screening and to avoid carcinogenicity of imaging technologies it is desirable to combine LDCT screening with sensitive blood biomarker testing.

Proliferation of malignant cells of lung cancer as of other cancers is driven by 'driver' mutations, due to disintegration of malignant cells, mutant cellular DNA is released to the circulation and can be detected via sensitive sequencing technologies. The technology has been labelled as liquid biopsy (LB). Technical review of sensitivity and specificity of LB indicates today less than necessary accuracy for efficient complementation of imaging for early detection of LC, partly due to fact that about 25% of LC has no characteristic mutation, and that small primary cancer nodules do not release sufficient quantity of mutant DNA for detection in regular blood sample volume.

Additionally, mutation of driver genes represents the root cause of the LC but it does not reflect actual status of cancer as much as multiplex panel based biomarker markers of protein nature. Here, we describe a proteome epitomics systems approach to detect and follow LC progression.

Global proteome analysis has been hampered by a variety of methodological problems including the fact that current mass spectrometry-based proteome profiling technologies are in general far from covering the necessary dynamic range and granularity; and are not adequately sensitive and lack sufficient reproducibility and throughput. Proteomics technologies focus on the global detection of representational differences of proteins exclusively. Most popular of these technologies are based on shot-gun mass spectrometry. Although, a global and hypothesis free approach, it provides only an estimate of protein levels. Moreover, standard forms of MS proteomics technologies do not address protein variants on a global scale. Affinity reagent based technologies like, recombinant antibody and SomaScan technologies (1,2), in principle may detect protein variants, however, reported studies focus on the detection of proteins at ultimately low levels rather than addressing protein variants on a global scale. This includes the Human Protein Atlas and other large scale initiatives, such as the NCI Clinical Proteomics Technology Initiative, which are targeted at the generation of a single mAb per protein (3). Differential screening of LC and control plasma protein epitome with complex nascent mAb libraries generated >50 candidates, however when translated and validated via sandwich ELISA assay detecting individual proteins the attrition was >90% (4). We now used the capture inhibition assay termed here CIA (5,6) that detects individual epitopes from biomarker candidate discovery to final validation. In the CIA a single capture mAb functions to detect and to monitor a single epitope in contrast to the sandwich assays, where due to the necessity of two binder reagents, single epitope monitoring at proteome scale is impossible.

So far, little effort has been dedicated to the development of proteomic systems approaches to monitor proteome variability and abundance simultaneously. Variants of proteins, among others, result from posttranslational modifications, alternative splicing, processing, degradation and complex formation, which all affect epitopes and protein function (7). In the current application, we demonstrate that extensive profiling of antigenic-epitome variation associated with cancer presents a novel source of valuable cancer specific biomarker panels.

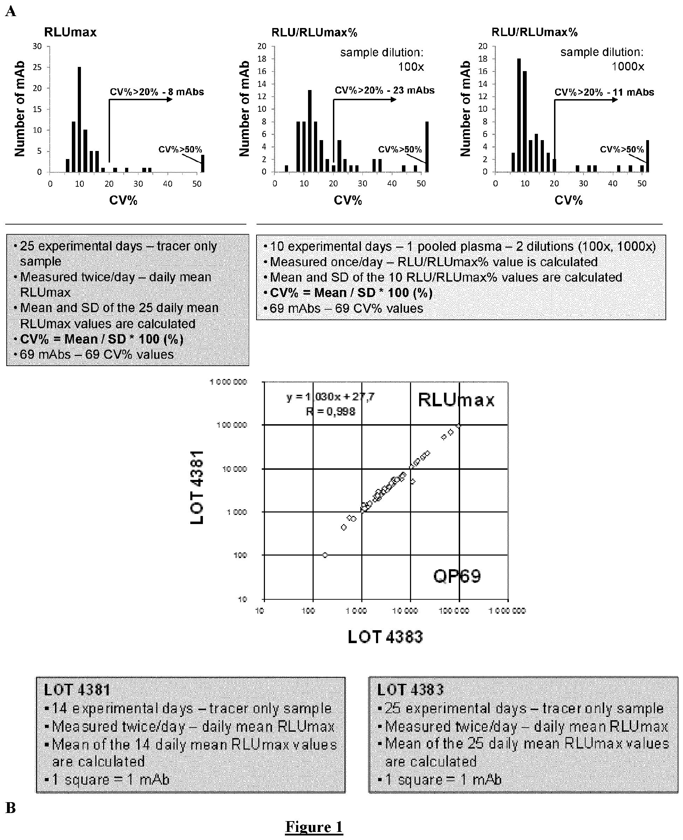

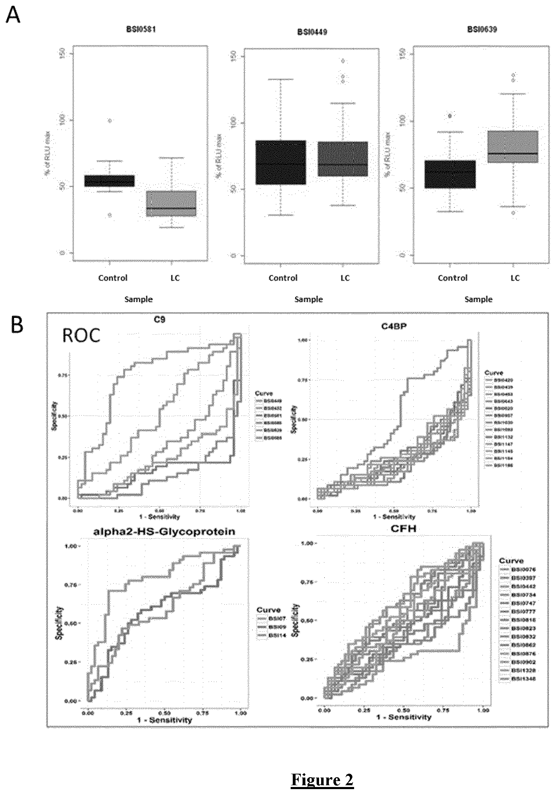

Biosystems inventors previously described hybridoma pool that has been generated against natural epitopes (8). This method was not precise with respect to detection of protein variability. To develop the present invention, the inventors tested individual cloned hybridoma derived mAb-s in the CIA assay in high throughput and automated 384 well ELISA and subjected the selected mAbs to analytical validation on the Randox Evidence Investigator platform (Figure 1). The inventors termed the library Quantiplasma™. Inventors, then used the Quantiplasma™ library and ran the assays from discovery through qualification and clinical validation on the Evidence Investigator instrument of Randox Ltd. and also on conventional 96w ELISA format. The inventors demonstrated that mAbs that recognize different cognate epitopes of the same protein provide markedly different level of association with lung cancer. This indicates that association of particular protein with a disease condition such as LC has to be determined at the level of protein epitopes via the determination of qualitative and quantitative features simultaneously (Figure 2).

WO2011/015602 discloses a method for detection of lung cancer involving determining the antigenic epitome associated with lung cancer.

EP2772759 discloses a method of diagnosing lung cancer involving detection of alpha-1-Antichymotrypsin, complement C9, Alpha-2-HS-Glycoprotein and C4bp as biomarkers.

## Summary of the invention

An abject of the invention relates to a method for detecting lung cancer in a subject, the method comprising contacting a sample from said subject, preferably a blood sample, with at least one antibody of the invention and determining the presence of an epitope-antigen bound to said at least one antibody, said presence being indicative of lung cancer.

Another object of the invention relates to a device for diagnosing, providing prognosis, monitoring, characterizing, determining a severity of, confirming a presence of, or confirming an absence of lung cancer in a subject.

## Detailed description of the invention

The claimed invention relates to a method as defined in independent claim 1 and a device as defined in independent claim 13. Preferred embodiments are defined in dependent claims 2-12.

All claimed embodiments involve at least the antibody panels defined in claim 1 and 13. Embodiments in the following which do not comprise at least these antibody panels are for reference only.

A first object of the disclosure relates to an *in vitro* or *ex vivo* method for detection / diagnosis / prognosis of lung cancer in a subject as defined above, wherein the step of determining the presence of an epitope-antigen bound to one antibody is performed by a capture inhibition assay.

More particularly an object of the invention is a method for detection of the probability of presence or absence of lung cancer (LC).

A particular object of the disclosure relates to an *in vitro* or *ex vivo* method for detection or prognosis of lung cancer (LC) in a subject, by determining the qualitative variation of the antigenic epitome associated with LC in a capture inhibition assay (CIA), the method comprising:

a) contacting a sample from said subject, preferably a blood plasma sample, with at least one antibody which binds an epitope-antigen comprising or consisting of a sequence selected from SEQ ID NO: 1 to 180, or one fragment or derivative of such an antibody having the same antigen specificity,
b) determining the presence or the absence of at least one epitope-antigen bound to said at least one antibody,
c) comparing the test results of the determination in step (b) to a control, whereby LC is to be detected / diagnosed / predicted, said presence or absence of an epitope-antigen bound to said at least one antibody being indicative of LC.

A particular object of the disclosure relates to an *in vitro* or *ex vivo* method for detection or prognosis of lung cancer (LC) in a subject, by determination of the qualitative and quantitative variation of the antigenic epitome associated with LC in a CIA assay, the method comprising:

a) contacting a sample from said subject, preferably a blood plasma sample, with at least one antibody which binds an epitope-antigen comprising or consisting of a sequence selected from SEQ ID NO: 1 to 180, or one fragment or derivative of such an antibody having the same antigen specificity,
b) determining the presence or the absence and the level of binding of at least one epitope-antigen bound to said at least one antibody,

c) comparing the test results of the determination in step (b) to a control, whereby LC is to be detected / diagnosed / predicted, said presence or absence of an epitope-antigen bound to said at least one antibody being indicative of LC.

According to the present invention, the expression "determining the level of binding of at least one epitope-antigen" means that during the CIA measurements, the results is expressed as RLU/RLUmax (RLU%), by determining (i) the maximal signal (RLUmax) that an epitope specific antibodies produces with the tracer (standard preparation of a protein mix from the plasma) and (ii) the signal (RLU) that an epitope specific antibody produces when the tested sample (i.e LC patient plasma) is mixed with the tracer. The semi quantitative measure of epitope level is derived from the two results by a simple division, RLU/RLUmax (RLU%). This method allows to determine differences but not in absolute measures (such as ng/L etc..).

A more particular object of the invention relates to a method that further comprises

d) calculating a probability index based on logistic regression models with forward or enter method; and

e) determining the probability of the presence or absence of LC in the subject, based on the probability index.

According to the present invention, the terms "probability index" and "risk indicator" of lung cancer are interchangeable and are calculated using the formulas $\hat{P}$ constructed from CIA measurement data via biochip and ELISA.

An even more particular object of the invention relates to a method that further comprises determining the level of binding of biomarkers chosen among the group consisting of albumin, CA125, CEA, CYFRA, TPAM, CRP, HE4 and CRP with known commercial kits.

The method preferably comprises contacting said sample from said subject with at least 2, 3, 4, 5, 6, 7, 8, 9, 10, 11, 12, 13, 14, 15, 16, 17, 18, 19, 20, 21 or 22 antibodies selected from the monoclonal antibodies Bsi0097, Bsi0116, Bsi0142, Bsi0144, Bsi0186, Bsi0190, Bsi0214, Bsi0221, Bsi0782, Bsi0300, Bsi0439, Bsi0581, Bsi0585, Bsi0639, Bsi0686, Bsi0759, Bsi0789, Bsi1154, Bsi1186, Bsi1328, Bsi1517 and Bsi2487 in combination.

Altogether, the disclosure thus provides novel antibodies which represent highly valuable reagents for cancer detection, management, diagnosis, monitoring, histopathological classification, staining, or imaging.

In a particular embodiment, the disclosure therefore relates to an antibody which binds an epitope-antigen having a sequence selected from SEQ ID NOs: 1 to 180 (see Table 1 below), or a fragment or derivative of such an antibody having the same antigen specificity.

Table 1

| mAb name | Protein ID | SEQ ID NO | Epitope-antigen sequences |
|---|---|---|---|
| Bsi0097 | albumin | 1 | NLPSVFKHLTSA |
| | | 2 | NLPNAWKHLTSA |
| | | 3 | LPQAAW KW LSSA |
| | | 4 | IPWSTTKWLSSA |
| | | 5 | TSFNLTKHLSSA |
| Bsi0116 | Alpha-1B-glycoprotein | 6 | YSPSAPGWPPMQ |
| | | 7 | SIEQPSGLSHAG |
| | | 8 | HSHTSAHHSTLT |
| | | 9 | EALNTKLCMACP |
| | | 10 | VCADHANYGRSR |
| | | 11 | TAAYHAINGGVN |
| | | 12 | NTPYLHMYRSIP |
| | | 13 | LPLQPPLASSPV |
| Bsi0144 | Alpha-1-antichymotrypsin | 14 | |
| | | | TPSPWTLVMLDH |
| | | 15 | AELSIRWWMDQL |
| | | 16 | MDVYTWQLLHAP |

(continued)

| mAb name | Protein ID | SEQ ID NO | Epitope-antigen sequences |
|---|---|---|---|
| Bsi0186 | Alpha-1-antichymotrypsin | 17 | DIWSTMEHNKYN |
| | | 18 | SVPWWTQSLLMS |
| | | 19 | HINAIQYPLPHT |
| Bsi0190 | complement C3 | 20 | SHSPWNISHLIR |
| | | 21 | ANFHAMNFQPPS |
| | | 22 | NGLHSPWMISTL |
| | | 23 | GTHAPWALWHFT |
| | | 24 | GSHDPIGLWLRF |
| | | 25 | HDPETLALLYPP |
| | | 26 | HKYQEPPWLLPG |
| Bsi0214 | ApoB100 | 27 | AYQFPRSNLWTP |
| | | 28 | VNPDYPRLNDWP |
| | | 29 | GPFMMMRMNTWP |
| | | 30 | SLTYPRLNSWSS |
| | | 31 | IAYHYPPLNSWG |
| | | 32 | APYYPYHMNQWQ |
| | | 33 | QLNYYYALNRWP |
| Bsi0221 | Alpha-1-antichymotrypsin | 34 | HEPMTGPKTNMQ |
| | | 35 | YTNPMWTLLQLR |
| | | 36 | HERMTGPKTNMQ |
| | | 37 | DSGPTQAPITPS |
| | | 38 | THLDGPPQSSEP |
| | | 39 | SHSSGAYRAPGE |
| | | 40 | NANFSSPGATST |
| | | 41 | VWATSPQQYRGH |
| | | 42 | IDRPVGSPVTSA |
| | | 43 | PSFQDYQLQSAD |
| | | 44 | SPSFSNKLPTGT |
| | | 45 | LPPHDTAIPAST |
| | | 46 | HPSETTDYSQRT |
| | | 47 | HIQSTHTLEKPL |
| | | 48 | KPQNHDGLNLPF |
| Bsi0300 | ApoB100 | 49 | WHWNAWNWSSQQ |
| | | 50 | TALQDTYVAYKQ |
| | | 51 | WHWGQPYWLVPA |
| | | 52 | WHRHWYSAPDVQ |
| | | 53 | WPLWHWQSLSNY |
| | | 54 | AGPSWKHWVGFT |
| | | 55 | TVLADVYIPYHT |
| | | 56 | TSHEPPAELWMR |
| | | 57 | THPNKGPVWKVS |
| | | 58 | QSGPTWKTHFPY |
| | | 59 | FPSWMHAWYSFQ |

(continued)

| mAb name | Protein ID | SEQ ID NO | Epitope-antigen sequences |
|----------|-----------|-----------|---------------------------|
| Bsi0439 | complement C4BP | 60 | THWYTWTSYYEY |
|  |  | 61 | GWYIQPTVRVKE |
|  |  | 62 | QVWYYSSSNEDL |
|  |  | 63 | DQYLYTTELVEL |
|  |  | 64 | HDRVTYSFTIQT |
|  |  | 65 | TTIITQLFLFDD |
|  |  | 66 | SLWHTFTTYIPI |
|  |  | 67 | SEFYYYETYMSY |
|  |  | 68 | NLWWMLGYNNSD |
|  |  | 69 | WHWSFNKTWASA |
|  |  | 70 | SYTYYTYYTETH |
|  |  | 71 | FHWYHPKPWYVN |
| Bsi0581 | complement C9 | 72 | IAGEYTWRYYTE |
|  |  | 73 | FPDPMGTWRYFQ |
|  |  | 74 | SVDPYATWRYRL |
|  |  | 75 | AVSGCRHAPSCT |
|  |  | 76 | NADQRCWEGRCK |
|  |  | 77 | NCLYPCAGNMLG |
| Bsi0585 | complement C9 | 78 | SGKQMIPHNQWP |
|  |  | 79 | LDGLSTWRFYR |
|  |  | 80 | GPVTLQELFQPW |
|  |  | 81 | GTPREGYHTMIS |
|  |  | 82 | ATRIPYAVANTP |
| Bsi0639 | complement C9 | 83 | EDFIPYFTHLNY |
|  |  | 84 | MPMYSALYTTP |
|  |  | 85 | LPPMYTPSYEHP |
|  |  | 86 | GTPREGYHTMIS |
|  |  | 87 | NSSPVSLPLLTF |
|  |  | 88 | VPPLYSSYHVPL |
|  |  | 89 | MPVMLPMYTDVA |
|  |  | 90 | TQQSGDWMPLLT |
|  |  | 91 | LPLAPHMVPYMT |
|  |  | 92 | TMPMLSRAGMLA |
|  |  | 93 | YSKPSAAQMTIL |
|  |  | 94 | TDILRAPLYTGA |
|  |  | 95 | GVSGPFDGGYLT |

(continued)

| mAb name | Protein ID | SEQ ID NO | Epitope-antigen sequences |
|---|---|---|---|
| Bsi0686 | complement C9 | 96 | VAFPLYVHSALV |
| | | 97 | NLAVESIFPLNA |
| | | 98 | QTPPPYMTTTVK |
| | | 99 | EPHLAPYRTGWT |
| | | 100 | NDQNIIMPFMTS |
| | | 101 | YMPLLTGLHLKN |
| | | 102 | SNSIPSPYLTYW |
| | | 103 | SHMTNQAPYLTQ |
| | | 104 | HPSLTQVPPYFT |
| | | 105 | IPPRLTPGFQVL |
| | | 106 | YMPLVVPHWLDP |
| | | 107 | NYHSNWWSTDTR |
| | | 108 | NYEMPYNTHWGL |
| Bsi0759 | alpha-2-HS-glycoprotein | 109 | DWYIRNTELIPI |
| | | 110 | CPDGMCRGNYIL |
| | | 111 | HIITYSFSISGT |
| | | 112 | GWVVTTELMPMG |
| | | 113 | GQTWYIYTDNRY |
| | | 114 | DRLNLTHFWVPH |
| Bsi0782 | complement C5 | 115 | WHWNAWNWSSQQ |
| | | 116 | WHWKFLNTNMPY |
| | | 117 | HVLSRPSTPADL |
| | | 118 | SHWWTRWNALAL |
| | | 119 | SHGRTRWNAQAP |
| | | 120 | IPWDHPNHVADK |
| | | 121 | WWSPYNARPTLG |
| | | 122 | TTWPTVHGQTRL |
| | | 123 | SHWSWFYRTVDS |
| | | 124 | AHPPKAPEPHKT |
| | | 125 | WHYPRLHSWFTQ |
| | | 126 | RPVESDPPLALQ |
| Bsi0789 | ApoB100 | 127 | THVYQNPGRHQA |
| | | 128 | SLNSPPPKYLAT |
| | | 129 | TWIWNLPPAPRG |
| | | 130 | ISNLGDTVTMVG |
| | | 131 | ISNLGYTVTMVG |
| | | 132 | NQWLHSTQYIYE |
| | | 133 | FHWPHIHSYWLA |
| | | 134 | WHFTWWVDNRMT |
| | | 135 | HTHTPNNGRFLP |
| Bsi1154 | complement C4BP | 136 | MPTDKDWEIMLK |
| | | 137 | LMPTDEWWYLKT |
| | | 138 | IPCYFGPPWCQR |
| | | 139 | YHNDDHWTLMLK |
| | | 140 | AFPTDLDWASWH |
| | | 141 | FVPTDSDWMRML |

(continued)

| mAb name | Protein ID | SEQ ID NO | Epitope-antigen sequences |
|---|---|---|---|
| Bsi1186 | complement C4BP | 142 | MEVNLEHIIYVS |
| | | 143 | MEVNLEWITHQP |
| | | 144 | MDINLEYQLLHR |
| | | 145 | APNIEKFFYLGH |
| | | 146 | METNLETYWRIE |
| | | 147 | TACDAMCEDTLK |
| Bsi1328 | Factor H | 148 | ESLVTDKLPKPR |
| | | 149 | WHWRWTPPDHFI |
| | | 150 | ELVTDKWPKWGT |
| | | 151 | EVVTDRWPKPSP |
| | | 152 | ESLATDKLPKPR |
| | | 153 | ESQVTDKLPKPR |
| | | 154 | EDVLVTDKIPKI |
| Bsi1517 | desmoplakin | 155 | DRLNLTHFWVPH |
| | | 156 | ALPLFFWEARAG |
| | | 157 | FLDVYGPKTLHF |
| | | 158 | GTLATHFWTTA |
| | | 159 | LCLPNSCKLQFD |
| | | 160 | LGRPGAPNPTWY |
| | | 161 | NFLDLYPPGLAA |
| | | 162 | NFTAPSAFYHWW |
| | | 163 | NGWIGPLIFDTS |
| | | 164 | NGWLGVLNVLPD |
| | | 165 | NPWILGLSAPSS |
| | | 166 | NPWLNDLATISY |
| | | 167 | QPTLRNPFVPMT |
| | | 168 | SLERWIDGLESL |
| | | 169 | TPTEMWLGRNFH |
| | | 170 | TSSLFPNIYGVK |
| | | 171 | VTYDKQFFWQEV |
| | | 172 | YVTHFWHRELPS |
| Bsi2487 | Gelsolin | 173 | ESEFALYMKYLY |
| | | 174 | SEYVQYLRFLGI |
| | | 175 | TLSEFELYLLTL |
| | | 176 | QSEASLYLKLVK |
| | | 177 | VSEFWWWDRLTM |
| | | 178 | AWSEYSLYKRFM |
| | | 179 | DVSGKWLKLLTL |
| | | 180 | DYHDPSLPTLRK |

In a particular embodiment, the disclosure relates to a method wherein the said at least one antibody is selected from:

- an antibody which binds an epitope-antigen comprising or consisting of a sequence selected from SEQ ID NO: 1 to 5, and which also binds a human serum albumin,
- an antibody which binds an epitope-antigen comprising or consisting of a sequence selected from SEQ ID NO: 6 to 13, and which also binds a human alpha-1B-glycoprotein,
- an antibody which binds an epitope-antigen comprising or consisting of a sequence selected from SEQ ID NO: 14 to 16, and which also binds a human alpha-1-antichymotrypsin protein,

- an antibody which binds an epitope-antigen comprising or consisting of a sequence selected from SEQ ID NO: 17 to 19, and which also binds a human alpha-1-antichymotrypsin protein,
- an antibody which binds an epitope-antigen comprising or consisting of a sequence selected from SEQ ID NO: 20 to 26, and which also binds a human complement C3 protein,
- an antibody which binds an epitope-antigen comprising or consisting of a sequence selected from SEQ ID NO: 27 to 33, and which also binds a human apolipoprotein B100 protein,
- an antibody which binds an epitope-antigen comprising or consisting of a sequence selected from SEQ ID NO: 34 to 48, and which also binds a human alpha-1-antichymotrypsin protein,
- an antibody which binds an epitope-antigen comprising or consisting of a sequence selected from SEQ ID NO: 49 to 59, and which also binds a human apolipoprotein B100 protein,
- an antibody which binds an epitope-antigen comprising or consisting of a sequence selected from SEQ ID NO: 60 to 71, and which also binds a human complement C4Bp protein,
- an antibody which binds an epitope-antigen comprising or consisting of a sequence selected from SEQ ID NO: 72 to 77, and which also binds a human complement C9 protein,
- an antibody which binds an epitope-antigen comprising or consisting of a sequence selected from SEQ ID NO: 78 to 82, and which also binds a human complement C9 protein,
- an antibody which binds an epitope-antigen comprising or consisting of a sequence selected from SEQ ID NO: 83 to 95, and which also binds a human complement C9 protein,
- an antibody which binds an epitope-antigen comprising or consisting of a sequence selected from SEQ ID NO: 96 to 108, and which also binds a human complement C9 protein,
- an antibody which binds an epitope-antigen comprising or consisting of a sequence selected from SEQ ID NO: 109 to 114, and which also binds a human alpha-2-HS-glycoprotein,
- an antibody which binds an epitope-antigen comprising or consisting of a sequence selected from SEQ ID NO: 115 to 126, and which also binds a human complement C5 protein,
- an antibody which binds an epitope-antigen comprising or consisting of a sequence selected from SEQ ID NO: 127 to 135, and which also binds a human apolipoprotein B100 protein,
- an antibody which binds an epitope-antigen comprising or consisting of a sequence selected from SEQ ID NO: 136 to 141, and which also binds a human complement C4Bp protein,
- an antibody which binds an epitope-antigen comprising or consisting of a sequence selected from SEQ ID NO: 142 to 147, and which also binds a human complement C4Bp protein,
- an antibody which binds an epitope-antigen comprising or consisting of a sequence selected from SEQ ID NO: 148 to 154, and which also binds the human factor H protein,
- an antibody which binds an epitope-antigen comprising or consisting of a sequence selected from SEQ ID NO: 155 to 172, and which also binds a human desmoplakin, and
- an antibody which binds an epitope-antigen comprising or consisting of a sequence selected from SEQ ID NO: 173 to 180, and which also binds a human gelsolin protein.

In a particular embodiment, the disclosure relates to a method wherein the said at least one antibody is an antibody which binds an epitope-antigen comprising or consisting of a sequence selected from SEQ ID NO: 72 to 77, and particularly the monoclonal antibody Bsi0581.

In a particular embodiment, the disclosure relates to a method wherein at least one antibody which binds said epitope-antigen is selected from the monoclonal antibodies: Bsi0097, Bsi0116, Bsi0142, Bsi0144, Bsi0186, Bsi0190, Bsi0214, Bsi0221, Bsi0300, Bsi0439, Bsi0581, Bsi0585, Bsi0639, Bsi0686, Bsi0759, Bsi0782, Bsi0789, Bsi1154, Bsi1186, Bsi1328, Bsi1517 and Bsi2487, or a fragment or derivative thereof having the same antigen specificity.

In a particular embodiment, the disclosure relates to a method wherein the step of determining the presence or absence and determining the said level of binding of at least one epitope-antigen comprises determining the level of binding of at least one epitope-antigen comprising or consisting of a sequence selected from SEQ ID NO: 1 to 180, and particularly at least one epitope-antigen of the following set of sequences: SEQ ID NO: 1 to 5, SEQ ID NO: 6 to 13, SEQ ID NO: 14 to 16, SEQ ID NO: 17 to 19, SEQ ID NO: 20 to 26, SEQ ID NO: 27 to 33, SEQ ID NO: 34 to 48, SEQ ID NO: 49 to 59, SEQ ID NO: 60 to 71, SEQ ID NO: 72 to 77, SEQ ID NO: 78 to 82, SEQ ID NO: 83 to 95, SEQ ID NO: 96 to 108, SEQ ID NO: 109 to 114, SEQ ID NO: 115 to 126, SEQ ID NO: 127 to 135, SEQ ID NO: 136 to 141, SEQ ID NO: 142 to 147, SEQ ID NO: 148 to 154, SEQ ID NO: 155 to 172 and SEQ ID NO: 173 to 180.

In a particular embodiment, the disclosure relates to a method wherein the step of determining the level of binding of at least one epitope-antigen comprises determining the level of binding of at least an epitope-antigen comprising or consisting of a sequence selected from SEQ ID NO: 72 to 77.

In a particular embodiment, the disclosure relates to a method wherein the step of determining said level of binding of at least one epitope-antigen comprises determining the level of binding of an epitope-antigen comprising or consisting of a sequence selected from SEQ ID NO: 72 to 77 and at least 1, 2, 3, 4, 5, 6, 7, 8, 9, 10, 11, 12, 13, 14, 15, 16 17, 18, 19, 20, or 21 epitope-antigen(s) of the following set of sequences: SEQ ID NO: 1 to 5, SEQ ID NO: 6 to 13, SEQ ID NO: 14 to 16, SEQ ID NO: 17 to 19, SEQ ID NO: 20 to 26, SEQ ID NO: 27 to 33, SEQ ID NO: 34 to 48, SEQ ID NO: 49 to 59, SEQ ID NO: 60 to 71, SEQ ID NO: 78 to 82, SEQ ID NO: 83 to 95, SEQ ID NO: 96 to 108, SEQ ID NO: 109 to 114, SEQ ID NO: 115 to 126, SEQ ID NO: 127 to 135, SEQ ID NO: 136 to 141, SEQ ID NO: 142 to 147, SEQ ID NO: 148 to 154, SEQ ID NO: 155 to 172 and SEQ ID NO: 173 to 180.

In a particular embodiment, the disclosure relates to a method wherein the step of determining the levels of binding of one or more epitope-antigen comprise determining the levels of binding of 22 epitope-antigens comprising or consisting of a sequence selected from SEQ ID NO: 1 to 180.

The method preferably comprises contacting said sample from said subject with at least one antibody as defined above, preferably at least 2, 3, 4, 5, 6, 7, 8, 9, 10, 11, 12, 13, 14, 15, 16 17, 18, 19, 20, 21, 22 in combination.

As disclosed in the examples, the specification discloses particular antibody combinations which allow specific and sensitive determination of LC in subjects.

These combinations include, without limitation:

- Bsi0144, Bsi0581, Bsi0186, Bsi2487, and Bsi0190
- Bsi0581, Bsi0759, Bsi0782, and Bsi1186
- Bsi0144, Bsi0581, Bsi2487, Bsi0116, Bsi0782, Bsi1186, Bsi0759, and Bsi0221
- Bsi0214, Bsi0221, Bsi0581, and Bsi1186

A preferred type of combination comprises Bsi0581 in combination with at least 2, 3, 4, 5, 6, 7, 8, 9, 10, 11, 12, 13, 14, 15, 16 17, 18, 19, 20, 21 additional antibodies.

A more particular object of the present invention concerns an *in vitro* or *ex vivo* method for detection or prognosis of lung cancer (LC) in a subject, by determination of the qualitative and quantitative variation of the antigenic epitome associated with LC, the method comprising:

a) contacting a sample from said subject, preferably a blood plasma sample, with at least five (5) antibodies wherein

- the first antibody specifically binds at least one epitope-antigen comprising or consisting of the sequences selected from the group consisting of SEQ ID NO: 14 to 16,
- the second antibody specifically binds at least one epitope-antigen comprising or consisting of the sequences selected from the group consisting of SEQ ID NO: 34 to 48,
- the third antibody specifically binds at least one epitope-antigen comprising or consisting of the sequences selected from the group consisting of SEQ ID NO: 72 to 77,
- the fourth antibody specifically binds at least one epitope-antigen comprising or consisting of the sequences selected from the group consisting of SEQ ID NO: 109 to 114, and
- the fifth antibody specifically binds at least one epitope-antigen comprising or consisting of the sequences selected from the group consisting of SEQ ID NO: 142 to 147,

or one fragment or derivative of such antibodies having the same antigen specificity,

b) determining the presence or the absence and the level of binding of at least the epitope-antigens of each group of sequences with said at least five antibodies, forming antibody/epitope-antigen complexes,

c) comparing the test results of the determination in step (b) to a control, whereby LC is to be detected / diagnosed / predicted, said presence or absence of the epitope-antigen complexes with said at least five antibodies being indicative of LC.

In another particular object of the invention the step a) of the method further comprises contacting a sample from said subject, preferably a blood plasma sample, with at least one (1) antibody which specifically binds at least one epitope-antigen comprising or consisting of the sequences selected from the group consisting of SEQ ID NO: 27 to 33, or one fragment or derivative of such an antibody having the same antigen specificity.

In another particular object of the invention the step a) of the method further comprises contacting a sample from said subject, preferably a blood plasma sample, with 15 other antibodies wherein each antibody specifically binds respectively at least one epitope-antigen of a single group comprising or consisting of the sequences selected from the groups consisting of SEQ ID NO: 1 to 5, SEQ ID NO: 6 to 13, SEQ ID NO: 17 to 19, SEQ ID NO: 20 to 26, SEQ ID NO: 49 to 59, SEQ ID NO: 60 to 71, SEQ ID NO: 78 to 82, SEQ ID NO: 83 to 95, SEQ ID NO: 96 to 108, SEQ ID NO: 115 to 126, SEQ ID NO: 127 to 135, SEQ ID NO: 136 to 141, SEQ ID NO: 148 to 154, SEQ ID NO: 155 to 172, and SEQ ID NO: 173 to 180, or one fragment or derivative of such an antibody having the same antigen specificity.

In another particular object of the invention the step a) of the method comprises contacting a sample from said subject, preferably a blood plasma sample, with six (6) antibodies wherein

- the first antibody specifically binds at least one epitope-antigen comprising or consisting of the sequences selected from the group consisting of SEQ ID NO: 14 to 16,
- the second antibody specifically binds at least one epitope-antigen comprising or consisting of the sequences selected from the group consisting of SEQ ID NO: 34 to 48,
- the third antibody specifically binds at least one epitope-antigen comprising or consisting of the sequences selected from the group consisting of SEQ ID NO: 72 to 77,
- the fourth antibody specifically binds at least one epitope-antigen comprising or consisting of the sequences selected from the group consisting of SEQ ID NO: 109 to 114,
- the fifth antibody specifically binds at least one epitope-antigen comprising or consisting of the sequences selected from the group consisting of SEQ ID NO: 142 to 147, and
- the sixth antibody specifically binds at least one epitope-antigen comprising or consisting of the sequences selected from the group consisting of SEQ ID NO: 27 to 33,

or one fragment or derivative of such antibodies having the same antigen specificity.

In another particular object of the invention the step a) of the method comprises contacting a sample from said subject, preferably a blood plasma sample, with twenty (20) antibodies wherein each antibody specifically binds respectively at least one epitope-antigen of a single group comprising or consisting of the sequences selected from the groups consisting of SEQ ID NO: 1 to 5, SEQ ID NO: 6 to 13, SEQ ID NO: 14 to 16, SEQ ID NO: 17 to 19, SEQ ID NO: 20 to 26, SEQ ID NO: 34 to 48, SEQ ID NO: 49 to 59, SEQ ID NO: 60 to 71, SEQ ID NO: 72 to 77, SEQ ID NO: 78 to 82, SEQ ID NO: 83 to 95, SEQ ID NO: 96 to 108, SEQ ID NO: 109 to 114, SEQ ID NO: 115 to 126, SEQ ID NO: 127 to 135, SEQ ID NO: 136 to 141, SEQ ID NO: 142 to 147, SEQ ID NO: 148 to 154, SEQ ID NO: 155 to 172, and SEQ ID NO: 173 to 180, or one fragment or derivative of such an antibody having the same antigen specificity.

Table 2 lists the antibodies and the epitopes-antigens SEQ ID NO involved in the QPLC96w and QPLC21 biochip measurement datasets.

Table 2

| mAb name | Protein ID | SEQ ID NO | QPLC21 | QPLC96w |
|---|---|---|---|---|
| Bsi0097 | albumin | 1-5 | + | - |
| Bsi0116 | Alpha-1B-glycoprotein | 6-13 | + | - |
| Bsi0144 | Alpha-1-antichymotrypsin | 14-16 | + | + |
| Bsi0186 | Alpha-1-antichymotrypsin | 17-19 | + | - |
| Bsi0190 | complement C3 | 20-26 | + | - |
| Bsi0214 | ApoB100 | 27-33 | - | + |
| Bsi0221 | Alpha-1-antichymotrypsin | 34-48 | + | + |
| Bsi0300 | ApoB100 | 49-59 | + | - |
| Bsi0439 | complement C4BP | 60-71 | + | - |
| Bsi0581 | complement C9 | 72-77 | + | + |
| Bsi0585 | complement C9 | 78-82 | + | - |
| Bsi0639 | complement C9 | 83-95 | + | - |
| Bsi0686 | complement C9 | 96-108 | + | - |
| Bsi0759 | alpha-2-HS-glycoprotein | 109-114 | + | + |
| Bsi0782 | complement C5 | 115-126 | + | - |
| Bsi0789 | ApoB100 | 127-135 | + | - |
| Bsi1154 | complement C4BP | 136-141 | + | - |
| Bsi1186 | complement C4BP | 142-147 | + | + |
| Bsi1328 | Factor H | 148-154 | + | - |
| Bsi1517 | desmoplakin | 155-172 | + | - |
| Bsi2487 | Gelsolin | 173-180 | + | - |

In another particular object of the invention, in step a) of the method

each antibody is immobilized directly to a support or captured by an affinity reagent such as an anti-mouse IgG antibody coated onto a support,

each immobilized antibody is then incubated with the sample from said subject.

In a particular embodiment, the method of the invention also comprises the measurement of one or more biomarkers chosen among the group consisting of albumin, CA125, CEA, CYFRA, TPAM, CRP, HE4 and CRP, preferably albumin, CRP and CYFRA, and their combinations.

In a particular embodiment, the method of the invention also comprises the measurement of the biomarker CYFRA.

In a particular embodiment of the invention the sample from a subject comprises a bodily fluid including but not limited to blood plasma, blood serum, thoracic inflammatory exudate.

In a particular embodiment, the invention relates to an *in vitro* or *ex vivo* method for detection / diagnosis / prognosis of lung cancer in a subject as defined above, wherein said subject has no symptoms of lung cancer.

In a particular embodiment, the invention relates to an *in vitro* or *ex vivo* method for detection / diagnosis / prognosis of lung cancer in a subject as defined above, wherein said subject has undergone surgery for the removal of lung cancer.

In a particular embodiment, the invention relates to an *in vitro* or *ex vivo* method for detection / diagnosis / prognosis of lung cancer in a subject as defined above, wherein said subject has at least one suspicious nodule in the lung.

For example, suspicious nodule in the lung can be detected by low-dose computed tomography (LDCT).

In a particular embodiment, the invention relates to an *in vitro* or *ex vivo* method for detection / diagnosis / prognosis of lung cancer in a subject as defined above, wherein said subject has at least one suspicious nodule in the lung, but no symptoms of lung cancer (LC).

In a particular embodiment, the invention relates to profile samples from subjects chosen among healthy subjects, stage I LC, stage II LC, stage III LC, stage IV LC, relapsing LC.

The invention also relates to an *in vitro* or *ex vivo* method for detection / diagnosis / prognosis / management of lung cancer in a subject, the method comprising:

a) contacting a sample from said subject, preferably a blood, plasma, serum sample, with at least one antibody as defined above,

b) determining the presence of an epitope-antigen bound to said at least one antibody,

c) calculating the risk indicator of lung cancer.

In particular, the disclosure relates to an *in vitro* or *ex vivo* method for detection / diagnosis / prognosis / management of lung cancer in a subject, the method comprising:

a) Contacting a sample from said subject, preferably a blood, plasma, serum sample, with at least one antibody as defined above,

b) Determining the presence of an epitope-antigen bound to said at least one antibody,

c) Calculating the risk indicator of lung cancer using the formulas constructed from biochip measurement data (see below)

## Description of the origin of the algorithms

Algorithms of multivariate models to determine lung cancer risks were determined by advanced machine learning and descriptive statistical approaches. The input data for statistical analysis included the variables shown in Table 3.

Table 3: List of variables used for statistical analysis

| Column name | Content | Explanation |
| --- | --- | --- |
| Albumin | g/l | Albumin level measured in plasma |
| CA125 | kIU/L | Cancer Antigen 125 level measured in plasma |
| CEA | $\mu$g/L | Carcinoembrionic antigen level measured in plasma |
| CYFRA | $\mu$g/L | CYFRA 21-1 level measured in plasma |
| TPAM | U/L | Tissue polypeptide antigen (TPA) level measured in plasma |

(continued)

| Column name | Content | Explanation |
|---|---|---|
| CRP | mg/L | C-reactive protein level determined from serum |
| HE4 | pmol/L | Human epididymis protein 4 determined from serum |
| BSI mAb name as BsiXXXX | RLUmax% values | mean of replicate RLUmax% values of the given BSI mAb spot obtained using QM504 or QP507 tracer on QPLC21 biochips. |
| QM_BSIXXXX | RLUmax% values | mean of replicate RLUmax% values of the given BSI mAb spot obtained using QM504 mixed tracer on QPLC21 biochips.* |
| QP_BSIXXXX | RLUmax% values | mean of replicate RLUmax% values of the given BSI mAb spot obtained using QP507 depleted tracer on QPLC21 biochips. * |
| BSI mAb name as BsiXXXX | ODmax% values | mean of replicate RLUmax% values of the given BSI mAb spot obtained using mixed tracer on ELISA experiments. |

According to the above explanation * "QM_" or "QP_" prefixes were used only on "QM504-Q.P507_mean" sheet to distinguish the data obtained with the different tracers over the same BSI mAb.

Input variables, only BSI variables or BSI variables in combination with all or a few selected conventional LC biomarkers and laboratory tests, were pre-sorted first by either statistical significance tests, Random Forest analysis, or Support Vector Machine with linear kernel function. In the next model building step, the first 5-10 variables of the sorted list were entered only. At this point there were two choices of the model building: Forward or Enter method.

The Forward method deploys variables added to a logistic regression formula one by one to that point until the model's performance is significantly improved.

The Enter method uses all of the variables (either selected by ranking lists derived from descriptive statistical analysis or machine learning statistical outputs) used in the definition of the logistic regression formula.

### Formulas constructed from biochip measurement data

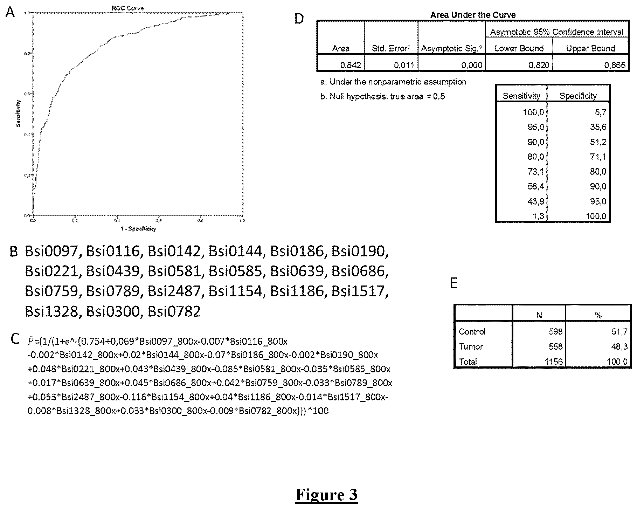

A formula (LC21) incorporating 21 epitomic variables provides remarkable discrimination as shown on Figure 3.

QPLC21 formula: $\hat{P}$=(1/(1+e^-(0.754+0,069*Bsi0097_800x-0.007*Bsi0116_800x - 0.002*Bsi0142_800x+0.02*Bsi0144_800x-0.07*Bsi0186_800x-0.002*Bsi0190_800x +0.048*Bsi0221_800x+0.043*Bsi0439_800x-0.085*Bsi0581_800x-0.035*Bsi0585_800x +0.017*Bsi0639_800x+0.045*Bsi0686_800x+0.042*Bsi0759_800x-0.033*Bsi0789_800x +0.053*Bsi2487_800x-0.116*Bsi1154_800x+0.04*Bsi1186_800x-0.014*Bsi1517_800x- 0.008*Bsi1328_800x+0.033*Bsi0300_800x-0.009*Bsi0782_800x))) *100

This model was built on the complete dataset of all QPLC21 epitomic BSI variables of the BD sample cohort (n=1156) measured with the 1:1 mixture of total and depleted tracer at 800x plasma dilution. During the statistical analysis variables used in Enter method to produce the formula.

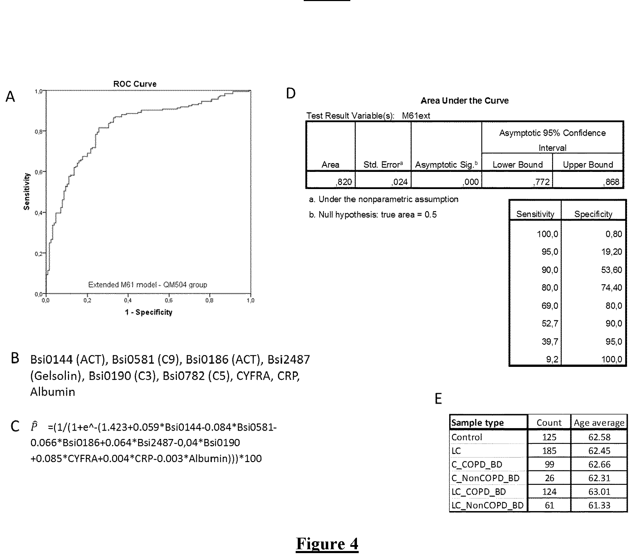

Further examples include Extended model 61 (Extended M61), Model 48 (M48), extended model 63 (Extended M63) which include conventional cancer biomarker, CYFRA and laboratory test data CRP and plasma albumin levels, which contribute to the performance of the epitomic biomarkers. Examples are shown on Figures 4-6. These three models were built on different subsets of the QPLC21 biochip measurement dataset of 310 matched sample set.

Extended M61 formula is derived from logistic regression analysis, by Enter method of manually selected BSI variables on the NSCLC/CE subpopulation of mixed tracer dataset.

Extended M61 formula: $\hat{P}$=(1/(1+e^-(1.423+0.059*Bsi0144-0.084*Bsi0581- 0.066*Bsi0186+0.064*Bsi2487-0,04*Bsi0190 +0.085*CYFRA+0.004*CRP-0.003*Albumin)))*100

M48 formula was developed by Forward method using preselected variables based on Mann-Whitney U test / independent t-test on QM504-QP507 NSCLC dataset.

$$\underline{\text{M48 formula: }} \hat{P}=(1/(1+e^\wedge(1{,}746+0{,}149*\text{CYFRA}-0{,}061*\text{QP\_Bsi0581}-0{,}056*\text{QP\_Bsi1186}+0{,}017*\text{QP\_Bsi0759}+0{,}045*\text{QP\_Bsi0782})))*100$$

Extended M63 formula is derived from logistic regression analysis, by Enter method of manually selected BSI variables of NSCLC/CE subpopulation of mixed and depleted tracer dataset. "QM_" prefix designates the data obtained with mixed tracer, while "QP_" with depleted tracer of the same BSI variable.

$$\underline{\text{Extended M63 formula: }} \hat{P}=(1/(1+e^\wedge(5.021+0.022*\text{QM\_Bsi0144}-0{,}071*\text{QP\_Bsi0581}$$
$$+0.026*\text{QP\_Bsi2487}-0{,}017*\text{QM\_Bsi0116}+0{,}006*\text{QP\_Bsi0782}$$
$$-0.019*\text{QM\_Bsi0581}-0{,}043*\text{QP\_Bsi0116}-0{,}027*\text{QM\_Bsi0186}$$
$$-0.025*\text{QP\_Bsi1186}+0.009*\text{QP\_Bsi0759}+0.052*\text{QP\_Bsi0221}$$
$$+0.080*\text{CYFRA}+0.004*\text{CRP}+0.009*\text{Albumin})))*100$$

96wQPLC6 M15 formula is derived from logistic regression analysis, by Forward method of BSI0144, BSI0214, BSI0221, BSI0581, BSI0759, BSI1186, Albumin, CA125, CEA, CYFRA, TPAM, CRP, HE4 variables on the BD early stage (LC stage I & II) lung cancer patient samples with controls having CYFRA measurement as well.

$$\underline{\text{96wQPLC6 M15 formula: }} P^\wedge = (1/(1 + e^\wedge(-(1{,}254 - 0{,}031*\text{BSI0214.1} + 0{,}088*\text{BSI0221.2} - 0{,}071*\text{BSI0581.2} - 0.060*\text{BSI1186.1} + 0{,}121*\text{CYFRA})))) * 100$$

96wQPLC6 M13 formula is derived from logistic regression analysis, by Forward method of BSI0144, BSI0214, BSI0221, BSI0581, BSI0759, BSI1186, Albumin, CA125, CEA, CYFRA, TPAM, CRP, HE4 variables on the BD late stage (LC stage III/B & IV) lung cancer patient samples with controls having CYFRA measurement as well.

$$\underline{\text{96wQPLC6 M13 formula: }} P^\wedge = (1/(1 + e^\wedge(-(0{,}105 - 0{,}037*\text{BSI0221.2} + 0.289*\text{CYFRA})))) * 100$$

96wQPLC6 M9 formula is derived from logistic regression analysis, by Enter method of BSI0144, BSI0214, BSI0221, BSI0581, BSI0759, BSI1186, CYFRA variables on the BD early stage (LC stage I, II & III/A) lung cancer patient samples with controls having CYFRA measurement as well.

$$\underline{\text{96wQPLC6 M9 formula: }} P^\wedge = (1/(1 + e^\wedge(-(1{,}721 - 0{,}002*\text{BSI0144.4} - 0{,}032*\text{BSI0214.1} + 0.059*\text{BSI0221.2} - 0.036*\text{BSI0581.2} + 0.010*\text{BSI0759.1} - 0.062*\text{BSI1186.1} + 0.156*\text{CYFRA})))) * 100$$

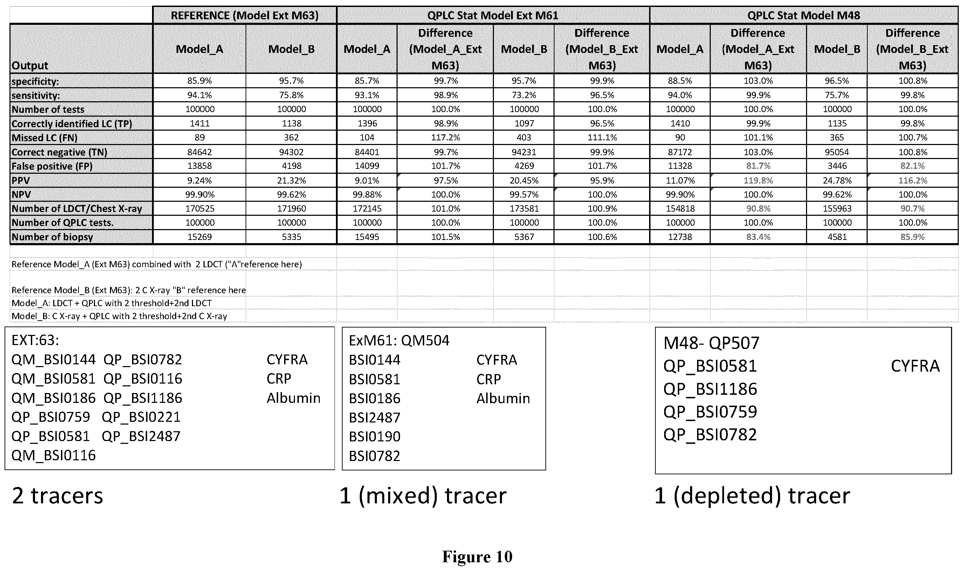

Performance data using Ext M63, Ext M61, M48, 96wQPLC6 M15, 96wQPLC6 M13, 96wQPLC6 M9 formula were combined (see later and methods) with data derived from published LC screening trials with low dose spiral computed tomography (LDCT) and chest X-ray performance in order to predict the value of epitomic biomarker panels if combined with standard imaging methodologies (10-12). The results are shown on Figure 10 and indicate specificity >85-95% with sensitivity > 95%-85% respectively for combination with LDCT or chest X-ray. The data predicts at least 50% decrease in the number of unnecessary diagnostic biopsies.

In a particular embodiment, the invention relates to an *in vitro* or *ex vivo* method for detection / diagnosis / prognosis of lung cancer in a subject as defined above, wherein the step of determining the presence of an epitope-antigen bound to one antibody is performed by a capture inhibition assay.

In particular embodiment the multivariate indices are generated from the individual epitomics variables with or without additional variables, such as known cancer biomarkers, laboratory, clinical and physiological parameters e.g. but not limited to plasma albumin and lung function test results.

Further object of the invention is the use of multivariate indices of the QPLC test in combination with imaging data such that differing thresholds derived from ROC analyses of statistical models of variables i.e., but not limited to Extended M63, extended M61, M48, 96wQPLC6 M15, 96wQPLC6 M13 and 96wQPLC6 M9 are used to orient subjects to the next step of diagnostic process. In this context the first, "high" threshold is determined to have high specificity such as 90%, 95% or above, and the second "low" threshold is determined to have high sensitivity such as 90%, 95% or above. Subjects are then categorized according to these thresholds, such as group I: "high" and above, this group has high probability of having lung cancer, group II: between "high" and "low" having medium level probability of lung cancer, and group III: at "low" or below "low" having low probability of lung cancer. Imaging, either X-ray examination or LDTC is administered in the first instance to all subjects, but during regular follow-up the imaging procedures are not administered at all to group III subjects or administered once every three years or less frequently, with yearly follow-up of blood testing. Group II patients will receive short term follow-up with Q.PLC test (3-6 month) and medium level frequency of imaging (once a year or one / two years) unless their Q.PLC test changes their position with respect to their group designation. Group I subjects will receive short term (3-6 months) follow-up with imaging and Q.PLC blood test if their results did not yet indicate immediate biopsy (>8 mm diameter solitaire nodule). With the strategy at the first combined use the QPLC test with imaging, our projections (Figure/Table 9) indicates substantial benefit, at least 50% reduction of unnecessary needle biopsies as compared to screening with imaging alone. Ultimately achieving a predicted sensitivity 75-95% with specificity 85-96% respectively, resulting in a predicted improvement of LC survival from 16% to 30-40%.

In a particular embodiment, the invention relates to a method that is able to discern subjects that do not have LC but have one or more symptoms associated with LC, from subjects having LC, with a receiver operator characteristic (ROC) AUC value of at least 0.75, preferably of at least 0.9.

In a particular embodiment, the invention relates to a method that is able to discern subjects not having LC but having one or more symptoms associated with LC, from subjects having LC, with a sensitivity of at least 80%, preferably of at least 85%, 90% or 95%.

In a particular embodiment, the invention relates to a method that is able to discern subjects not having LC but having one or more symptoms associated with LC, from subjects having LC, with specificity of at 80%, preferably of at least 85%, 90% or 95%.

## ANTIBODIES

In the following antibodies are disclosed, which can be used in the claimed invention but the antibodies per se are not claimed.

The antibodies of the invention derive from large epitome specific mAb libraries generated against the complex human plasma proteome of apparently healthy and cancer subjects. The advantage of the mAb libraries is that these mAbs react with natural epitopes.

The Inventors found that medium and high abundant protein targets can be used as disease specific biomarkers not at the protein level (quantitative variation in the proteome) but the epitope level (qualitative variation in the proteome).

The Inventors demonstrated that these protein epitope targets show significant representational level differences in between lung cancer patients and apparently healthy controls.

Binding epitope-antigens recognized by antibodies of the invention are disclosed below. These epitope-antigens have been tested in phage display ELISA and they also bind to the corresponding mAbs in biotinylated form, immobilized to avidin coated 96 well plates, in direct ELISA experiments.

In the invention, these epitope-antigens are also referred to as mimotop peptides because they assume three dimensional conformation states corresponding to cognate antigenic determinant sites on the surface of an antigen molecule to which a single antibody molecule binds.

Generally, an antigenic molecule has several or many different cognate antigenic determinants, termed as epitopes and these epitopes react with different mAbs. Consequently, the antigenic epitome variations offer a combined quantitative and qualitative approach to identify proteomic changes associated with disease or pre-disease states.

With respect to lung cancer the following epitopes are discriminatory in comparison to cancer free age and sex-matched subjects and together represent a lung cancer specific epitope panel:

Bsi0097, Bsi0116, Bsi0142, Bsi0144, Bsi0186, Bsi0190, Bsi0214, Bsi0221, Bsi0782, Bsi0300, Bsi0439, Bsi0581, Bsi0585, Bsi0639, Bsi0686, Bsi0759, Bsi0789, Bsi1154, Bsi1186, Bsi1328, Bsi1517, Bsi2487.

*Bsi0097*

In a particular embodiment, the invention relates to an antibody which binds an epitope-antigen comprising or consisting of a sequence selected from SEQ ID NO: 1 to 5, and which also binds a human serum albumin, or a fragment or derivative of such an antibody having the same antigen specificity. The invention particularly relates to an antibody which binds an epitope-antigen selected from SEQ ID NO: 1 to 5.

Using cDNA sequence of the variable IgG light and heavy chain variable regions, the crystal structure of albumin and SEQ NO: 1 to 5 epitope-antigens (peptides) present on the surface of human albumin has been determined, by structural modeling, the finding has been validated in biological experiments.

*Bsi0116*

In a particular embodiment, the invention relates to an antibody which binds an epitope-antigen comprising or consisting of a sequence selected from SEQ ID NO: 6 to 13, and which also binds a human alpha-1B-glycoprotein, or a fragment or derivative of such an antibody having the same antigen specificity. The invention particularly relates to an antibody which binds an epitope-antigen selected from SEQ ID NO: 6 to 13.

*Bsi0144*

In a particular embodiment, the invention relates to an antibody which binds an epitope-antigen comprising or consisting of a sequence selected from SEQ ID NO: 14 to 16, and which also binds a human alpha-1-antichymotrypsin protein, or a fragment or derivative of such an antibody having the same antigen specificity. The invention particularly relates to an antibody which binds an epitope-antigen selected from SEQ ID NO: 14 to 16.

*Bsi0186*

In a particular embodiment, the invention relates to an antibody which binds an epitope-antigen comprising or consisting of a sequence selected from SEQ ID NO: 17 to 19, and which also binds a human alpha-1-antichymotrypsin protein, or a fragment or derivative of such an antibody having the same antigen specificity. The invention particularly relates to an antibody which binds an epitope-antigen selected from SEQ ID NO: 17 to 19.

*Bsi0190*

In a particular embodiment, the invention relates to an antibody which binds an epitope-antigen comprising or consisting of a sequence selected from SEQ ID NO: 20 to 26, and which also binds a human complement C3 protein, or a fragment or derivative of such an antibody having the same antigen specificity. The invention particularly relates to an antibody which binds an epitope-antigen selected from SEQ ID NO: 20 to 26.

*Bsi0214*

In a particular embodiment, the invention relates to an antibody which binds an epitope-antigen comprising or consisting of a sequence selected from SEQ ID NO: 27 to 33, and which also binds a human apolipoprotein B100 protein, or a fragment or derivative of such an antibody having the same antigen specificity. The invention particularly relates to an antibody which binds an epitope-antigen selected from SEQ ID NO: 27 to 33.

*Bsi0221*

In a particular embodiment, the invention relates to an antibody which binds an epitope-antigen comprising or consisting of a sequence selected from SEQ ID NO: 34 to 48, and which also binds a human alpha-1-antichymotrypsin protein, or a fragment or derivative of such an antibody having the same antigen specificity. The invention particularly relates to an antibody which binds an epitope-antigen selected from SEQ ID NO: 34 to 48.

*Bsi0300*

In a particular embodiment, the invention relates to an antibody which binds an epitope-antigen comprising or consisting of a sequence selected from SEQ ID NO: 49 to 59, and which also binds a human apolipoprotein B100 protein, or a fragment or derivative of such an antibody having the same antigen specificity. The invention particularly relates to an antibody which binds an epitope-antigen selected from SEQ ID NO: 49 to 59.

*Bsi0439*

In a particular embodiment, the invention relates to an antibody which binds an epitope-antigen comprising or consisting of a sequence selected from SEQ ID NO: 60 to 71, and which also binds a human complement C4Bp protein, or a fragment or derivative of such an antibody having the same antigen specificity. The invention particularly relates to an antibody which binds an epitope-antigen selected from SEQ ID NO: 60 to 71.

*Bsi0581*

In a particular embodiment, the invention relates to an antibody which binds an epitope-antigen comprising or consisting of a sequence selected from SEQ ID NO: 72 to 77, and which also binds a human complement C9 protein, or a fragment or derivative of such an antibody having the same antigen specificity. The invention particularly relates to an antibody which binds an epitope-antigen selected from SEQ ID NO: 72 to 77.

*Bsi0585*

In a particular embodiment, the invention relates to an antibody which binds an epitope-antigen comprising or consisting of a sequence selected from SEQ ID NO: 78 to 82, and which also binds a human complement C9 protein, or a fragment or derivative of such an antibody having the same antigen specificity. The invention particularly relates to an antibody which binds an epitope-antigen selected from SEQ ID NO: 78 to 82.

*Bsi0639*

In a particular embodiment, the invention relates to an antibody which binds an epitope-antigen comprising or consisting of a sequence selected from SEQ ID NO: 83 to 95, and which also binds a human complement C9 protein, or a fragment or derivative of such an antibody having the same antigen specificity. The invention particularly relates to an antibody which binds an epitope-antigen selected from SEQ ID NO: 83 to 95.

*Bsi0686*

In a particular embodiment, the invention relates to an antibody which binds an epitope-antigen comprising or consisting of a sequence selected from SEQ ID NO: 96 to 108, and which also binds a human complement C9 protein, or a fragment or derivative of such an antibody having the same antigen specificity. The invention particularly relates to an antibody which binds an epitope-antigen selected from SEQ ID NO: 96 to 108.

*Bsi0759*

In a particular embodiment, the invention relates to an antibody which binds an epitope-antigen comprising or consisting of a sequence selected from SEQ ID NO: 109 to 114, and which also binds a human alpha-2-HS-glycoprotein, or a fragment or derivative of such an antibody having the same antigen specificity. The invention particularly relates to an antibody which binds an epitope-antigen selected from SEQ ID NO: 109 to 114.

*Bsi0782*

In a particular embodiment, the invention relates to an antibody which binds an epitope-antigen comprising or consisting of a sequence selected from SEQ ID NO: 115 to 126, and which also binds a human complement C5 protein, or a fragment or derivative of such an antibody having the same antigen specificity. The invention particularly relates to an antibody which binds an epitope-antigen selected from SEQ ID NO: 115 to 126.

*Bsi0789*

In a particular embodiment, the invention relates to an antibody which binds an epitope-antigen comprising or consisting of a sequence selected from SEQ ID NO: 127 to 135, and which also binds a human apolipoprotein B100 protein, or a fragment or derivative of such an antibody having the same antigen specificity. The invention particularly relates to an antibody which binds an epitope-antigen selected from SEQ ID NO: 127 to 135.

*Bsi1154*

In a particular embodiment, the invention relates to an antibody which binds an epitope-antigen comprising or consisting of a sequence selected from SEQ ID NO: 136 to 141, and which also binds a human complement C4b-binding protein (C4BP) protein, or a fragment or derivative of such an antibody having the same antigen specificity. The invention particularly relates to an antibody which binds an epitope-antigen selected from SEQ ID NO: 136 to 141.

*Bsi1186*

In a particular embodiment, the invention relates to an antibody which binds an epitope-antigen comprising or consisting of a sequence selected from SEQ ID NO: 142 to 147, and which also binds a human complement C4b-binding protein (C4BP) protein, or a fragment or derivative of such an antibody having the same antigen specificity. The invention particularly relates to an antibody which binds an epitope-antigen selected from SEQ ID NO: 142 to 147.

*Bsi1328*

In a particular embodiment, the invention relates to an antibody which binds an epitope-antigen comprising or consisting of a sequence selected from SEQ ID NO: 148 to 154, and which also binds the human factor H protein, or a fragment or derivative of such an antibody having the same antigen specificity. The invention particularly relates to an antibody which binds an epitope-antigen selected from SEQ ID NO: 148 to 154.

*Bsi1517*

In a particular embodiment, the invention relates to an antibody which binds an epitope-antigen comprising or consisting of a sequence selected from SEQ ID NO: 155 to 172, and which also binds a human desmoplakin protein, or a fragment or derivative of such an antibody having the same antigen specificity. The invention particularly relates to an antibody which binds an epitope-antigen selected from SEQ ID NO: 155 to 172.

*Bsi2487*

In a particular embodiment, the invention relates to an antibody which binds an epitope-antigen comprising or consisting of a sequence selected from SEQ ID NO: 173 to 180, and which also binds a human gelsolin protein, or a fragment or derivative of such an antibody having the same antigen specificity. The invention particularly relates to an antibody which binds an epitope-antigen selected from SEQ ID NO: 173 to 180.

Ilustrative and preferred examples of antibodies of the invention include monoclonal antibodies Bsi0097, Bsi0116, Bsi0142, Bsi0144, Bsi0186, Bsi0190, Bsi0214, Bsi0221, Bsi0782, Bsi0300, Bsi0439, Bsi0581, Bsi0585, Bsi0639, Bsi0686, Bsi0759, Bsi0789, Bsi1154, Bsi1186, Bsi1328, Bsi1517 and Bsi2487, or a fragment or derivative thereof having the same antigen specificity.

The cognate antigens for these 22 monoclonal antibodies have been identified, as well as binding sequences thereof. One mAb is specific for albumin, four are specific for alpha-1-antichymotrypsin, one is specific for alpha-1B-glycoprotein, one is specific for alpha-2-HS-glycoprotein, one is specific for complement C3, three are specific for complement C4BP, one is specific for complement C5, four are specific for complement C9, one is specific for factor H, one is specific for Gelsolin protein, three are specific for apolipoprotein B100 and one is specific for desmoplakin.

As mentioned above, the invention relates to antibodies (or fragments or derivatives thereof) which "bind" epitopes of human blood plasma proteins, herein the epitopes are defined by peptides, thus mimotops. One skilled in the arts is familiar with the terminology and the technology of mimotop definition. In the context of the invention, such binding should be specific or selective, meaning that the binding to the epitope-antigens of the present invention can be discriminated from (e.g., occurs with higher affinity or avidity than) possible nonspecific binding to other antigens.

Each antibody in the invention binds a single blood plasma protein specifically. Such antibodies can bind a variety of epitope-antigens. Thus, such an antibody can bind one, many or all epitope-antigens (peptides) from a group of specific epitope-antigens of the present invention.

Binding of an antibody to the epitope-antigens of the present invention can be tested with methods well known for one skilled in the art. Binding to an epitope-antigen can be verified with either the isolated peptide (e.g., immobilized on a support) or with the epitope-antigen included in a larger polypeptide sequence. In a particular embodiment, the epitope-antigen (peptide) is in isolated form and immobilized on a support (e.g., a plate) and the candidate antibody is incubated with the immobilized epitope-antigen (peptide). Binding may then be revealed using known techniques. Binding to a protein may be tested by incubating any sample containing the protein in solution, and verifying the formation of an immune complex. In a particular embodiment, the term "binding" to an epitope-antigen (peptide) indicates the antibody can bind the corresponding epitope-antigen (peptide) in biotinylated form, immobilized to avidin coated well plates in direct ELISA experiments.

The antibody may be a polyclonal or a monoclonal antibody, most preferably a monoclonal antibody. It may be of various classes (e.g., IgG, IgE, IgM, etc.). The antibody may be of various animal origin, or human or synthetic or recombinant. Furthermore, the term antibody also includes fragments and derivatives thereof, in particular fragments and derivatives of said monoclonal or polyclonal antibodies having substantially the same antigenic specificity. Antibody fragments include e.g., Fab, Fab '2, CDRs, etc). Derivatives include humanized antibodies, human antibodies, chimeric antibodies, poly-functional antibodies, Single Chain antibodies (ScFv), etc. These may be produced according to conventional methods, including immunization of an animal and collection of serum (polyclonal) or spleen cells (to produce hybridomas by fusion with appropriate cell lines).

Polyclonal antibodies may be obtained from various species, including mice, rodents, primates, horses, camels, sheep, pigs, rabbits, poultry, using conventional methods well known per se in the art. Briefly, the antigen is combined with an adjuvant (e.g., Freund's adjuvant) and administered to an animal, typically by sub-cutaneous injection. Repeated injections may be performed. Blood samples are collected and immunoglobulins or serum are separated.

Monoclonal antibodies from various species as listed above may be obtained using conventional methods well known per se in the art. Briefly, these methods comprise immunizing an animal with the antigen, followed by a recovery of spleen cells which are then fused with immortalized cells, such as myeloma cells. The resulting hybridomas produce the monoclonal antibodies and can be selected by limit dilutions or via automated micromanipulators, cloning robots to isolate individual clones. Antibodies may also be produced by selection of combinatorial or mutagenized libraries of recombinant immunoglobulins.

Recombinant antibodies of the invention, or fragments or derivatives thereof, may be produced by methods well known per se in the art, for example by recombination in a host cell, transformed with one or more vectors enabling the expression and/or secretion of the nucleotide sequences encoding the heavy chain or the light chain of the antibody or single-chain antibody versions. The vector generally contains a promoter, translation initiation and termination signals, and suitable transcriptional regulatory regions. It is stably maintained in the host cell and may optionally possess specific signals for secretion of the translated protein. These different components are selected and optimized by one of skill in the art according to the host cell used.

The antibodies of the invention may be coupled to heterologous moieties, such as toxins, labels, drugs or other therapeutic agents, covalently or not, either directly or through the use of coupling agents or linkers. Labels include radiolabels, enzymes, fluorescent labels, magnetic particles and the like. Toxins include diphtheria toxins, botulinum toxin, etc. Drugs or therapeutic agents include lymphokines, antibiotics, antisense RNA or antisense nucleic acid, modified or not, growth factors, etc.

The invention also relates to a composition comprising an antibody (or a fragment or derivative thereof) as defined above. The composition may comprise any excipient or solid support.

## NUCLEIC ACIDS

Another object of the disclosure is an isolated nucleic acid encoding an antibody of the invention, or the fragment or derivative of said antibody.

The nucleic acid typically encodes at least a portion of a variable region of the antibody, e.g., a portion of the heavy or light chain including a variable domain, such as a CDR or FR domain. The nucleic acid may be DNA or RNA, including cDNA, gDNA, recombinant DNA, synthetic or semisynthetic DNA, which may be single- or double-stranded.

A particular object of the disclosure is a nucleic acid encoding a polypeptide comprising at least a CDR or FR domain of an antibody as defined above. The nucleic acid may be fused to other regions (e.g., constant regions, hinge regions, etc.) to create synthetic antibodies, humanized antibodies, chimeric antibodies, etc., according to techniques well known per se in the art. They may also be used to produce single chain antibodies.

Another object of the disclosure is an expression vector, for example a viral or plasmid vector, comprising a nucleic acid of the invention.

The vector may replicate autonomously in the chosen host cell, or it may be an integrative vector. Also useful is an expression vector comprising a nucleic acid coding for the light chain or for the heavy chain of an antibody of the invention.

Such vectors are prepared by methods known per se in the art, and the resulting clones may be introduced into a suitable host cell by standard methods, such as lipofection, electroporation, use of polycationic agents, heat shock, or chemical methods.

## CELLS

Another object of the disclosure is a host cell transfected with said vector or vectors. The host cell may be selected from among prokaryotic or eukaryotic systems, for example bacterial cells but also yeast cells or animal cells, in particular mammalian cells. Insect cells or plant cells may also be used.

Another object of the disclosure is a hybridoma cell producing an antibody of the invention.

**METHODS**

In another aspect, the disclosure relates to a method for producing an antibody of the invention, said method comprising the following steps:

a) culturing in a suitable culture medium a host cell expressing a heavy chain and/or a light chain such as defined herein; and

b) recovering said antibodies so produced from the culture medium or from said cultured cells.

To allow the simultaneous expression of the heavy chain (H chain) and the light chain (L chain) and their re-association to form the recombinant antibody molecule, one may use two cassettes in a same expression vector. In this manner for example a double-recombinant vector may be prepared in which the sequence encoding each of the H and L chains is under the control of a strong promoter.

A particular example of a production method is production in an insect cell. To this end, an expression cassette is used comprising a sequence coding for the variable region of the monoclonal antibody light chain, or a sequence coding for the variable region of the monoclonal antibody heavy chain, said sequence is placed under transcriptional control of a suitable promoter, for example a baculovirus promoter.

Another example of a production method is the use of a viral or plasmid expression vector for expressing the monoclonal antibody in a mammalian cell. Preferred mammalian cells for expressing the monoclonal antibody are the rat YB2/0 line, the hamster CHO line, in particular the lines CHO dhfr- and CHO Led 3, PER.C6TM (Crucell), 293, K562, NSO, SP2/0, BHK or COS, COS7.

A further production method is the expression of the recombinant antibody in transgenic organisms, for example in plants or else in the milk of transgenic animals such as rabbit, goat or pig.

The antibodies of this disclosure have various applications, including, diagnostic, purification, detection, therapeutic, prophylactic, etc.

They can be used as screening agents or to purify the antigen from various samples, including various biological samples (e.g., samples of blood, plasma, serum, urine, sputum, cerebrospinal fluid, inflammatory exudate, such as synovial fluid and faces).

As demonstrated in the examples, these antibodies have the remarkable property of binding target proteins and detecting epitomic variation that are apparent via differential representation between cancer patients, particularly lung cancer patients and control subjects. In other words, the antibodies of the invention allow detection of the dynamic change of the epitome that correlates with the presence of lung cancer.

Thus, the disclosure relates to the use of the antibodies of the disclosure for the *ex vivo* or *in vitro* detection / diagnosis / prognosis / management of lung cancer.

As shown in the examples, the specification discloses that the particular combination of the 22 monoclonal antibodies Bsi0097, Bsi0116, Bsi0142, Bsi0144, Bsi0186, Bsi0190, Bsi0214, Bsi0221, Bsi0782, Bsi0300, Bsi0439, Bsi0581, Bsi0585, Bsi0639, Bsi0686, Bsi0759, Bsi0789, Bsi1154, Bsi1186, Bsi1328, Bsi1517 and Bsi2487 allows specific and sensitive determination of lung cancer (LC) in human subjects.

Another combination comprises Bsi0097 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0116 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0142 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0144 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0186 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0190 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0214 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0221 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0300 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0439 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0581 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0585 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0639 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0686 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0759 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0782 in combination with at 1, 2, 3, 4, 5, 6, 7, 8, 9, 10, 11, 12, 13, 14, 15, 16, 17, 18, 19, 20 or 21 additional antibodies.

Another combination comprises Bsi0789 in combination with at 1, 2, 3, 4, 5, 6, 7, 8, 9, 10, 11, 12, 13, 14, 15, 16, 17, 18, 19, 20 or 21 additional antibodies.

Another combination comprises Bsi1154 in combination with at 1, 2, 3, 4, 5, 6, 7, 8, 9, 10, 11, 12, 13, 14, 15, 16, 17, 18, 19, 20 or 21 additional antibodies.

Another combination comprises Bsi1186 in combination with at 1, 2, 3, 4, 5, 6, 7, 8, 9, 10, 11, 12, 13, 14, 15, 16, 17, 18, 19, 20 or 21 additional antibodies.

Another combination comprises Bsi1328 in combination with at 1, 2, 3, 4, 5, 6, 7, 8, 9, 10, 11, 12, 13, 14, 15, 16, 17, 18, 19, 20 or 21 additional antibodies.

Another combination comprises Bsi1517 in combination with at 1, 2, 3, 4, 5, 6, 7, 8, 9, 10, 11, 12, 13, 14, 15, 16, 17, 18, 19, 20 or 21 additional antibodies.

Another combination comprises Bsi2487 in combination with at 1, 2, 3, 4, 5, 6, 7, 8, 9, 10, 11, 12, 13, 14, 15, 16, 17, 18, 19, 20 or 21 additional antibodies.

The term "combination" indicates the sample should be tested for antigen binding to all antibodies of the combination, either simultaneously or separately (e.g., sequentially). Preferably, the antibodies are tested simultaneously (e.g., on the same device).

In particular embodiments, the disclosure relates to specific combinations of 2, 3, 4, 5, 6, 7, 8, 9, 10, 11, 12, 13, 14, 15, 16, 17, 18, 19, 20, 21 or 22 antibodies of the invention that can be used to detect a lung cancer in the presence of chronic obstructive pulmonary disease (COPD) or to detect a lung cancer in the absence of COPD.

In a particular embodiment, the invention relates to an *in vitro* or *ex vivo* method for detection / diagnosis / prognosis of lung cancer in a subject as defined above, wherein said subject is suspected of having a lung cancer.

DEVICES

The claimed invention relates to a device as defined in independent claim 13. Embodiments in the following which do not comprise at least the antibody panel as defined in claim 13 are for reference only.

A further object of the disclosure is a device comprising at least one antibody of the invention, or one fragment or derivative of such an antibody having the same epitope-antigen specificity.

In a particular embodiment, disclosed is a device comprising at least one antibody which binds an epitope-antigen (peptide) comprising or consisting of a sequence selected from SEQ ID NO: 1 to 180, or one fragment or derivative of such an antibody having the same antigen specificity.

In a particular embodiment, disclosed is a device comprising:

- an antibody which binds an epitope-antigen (peptide) comprising or consisting of a sequence selected from SEQ ID NO: 1 to 5, and
- at least one antibody which binds an epitope-antigen (peptide) comprising or consisting of a sequence selected from SEQ ID NO: 6 to 180.

In a particular embodiment, disclosed is a device comprising:

- an antibody which binds an epitope-antigen (peptide) comprising or consisting of a sequence selected from SEQ ID NO: 6 to 13, and
- at least one antibody which binds an epitope-antigen (peptide) comprising or consisting of a sequence selected from SEQ ID NO: 1 to 5 and SEQ ID NO: 14 to 180.

In a particular embodiment, disclosed is a device comprising:

- an antibody which binds an epitope-antigen (peptide) comprising or consisting of a sequence selected from SEQ ID NO: 14 to 16, and
- at least one antibody which binds an epitope-antigen (peptide) comprising or consisting of a sequence selected from SEQ ID NO: 1 to 13 and SEQ ID NO: 17 to 180.

In a particular embodiment, disclosed is a device comprising:

- an antibody which binds an epitope-antigen (peptide) comprising or consisting of a sequence selected from SEQ ID NO: 17 to 19, and
- at least one antibody which binds an epitope-antigen (peptide) comprising or consisting of a sequence selected from SEQ ID NO: 1 to 16 and SEQ ID NO: 20 to 180.

In a particular embodiment, disclosed is a device comprising:

- an antibody which binds an epitope-antigen (peptide) comprising or consisting of a sequence selected from SEQ ID NO: 20 to 26, and
- at least one antibody which binds an epitope-antigen (peptide) comprising or consisting of a sequence selected from SEQ ID NO: 1 to 19 and SEQ ID NO: 27 to 180.

In a particular embodiment, disclosed is a device comprising:

- an antibody which binds an epitope-antigen (peptide) comprising or consisting of a sequence selected from SEQ ID NO: 27 to 33, and
- at least one antibody which binds an epitope-antigen (peptide) comprising or consisting of a sequence selected from SEQ ID NO: 1 to 26 and SEQ ID NO: 34 to 180.

In a particular embodiment, disclosed is a device comprising:

- an antibody which binds an epitope-antigen (peptide) comprising or consisting of a sequence selected from SEQ ID NO: 34 to 48, and
- at least one antibody which binds an epitope-antigen (peptide) comprising or consisting of a sequence selected from SEQ ID NO: 1 to 33 and SEQ ID NO: 49 to 180.

In a particular embodiment, disclosed is a device comprising:

- an antibody which binds an epitope-antigen (peptide) comprising or consisting of a sequence selected from SEQ ID NO: 49 to 59, and
- at least one antibody which binds an epitope-antigen (peptide) comprising or consisting of a sequence selected from SEQ ID NO: 1 to 48 and SEQ ID NO: 60 to 180.

In a particular embodiment, disclosed is a device comprising:

- an antibody which binds an epitope-antigen (peptide) comprising or consisting of a sequence selected from SEQ ID NO: 60 to 71, and
- at least one antibody which binds an epitope-antigen (peptide) comprising or consisting of a sequence selected from SEQ ID NO: 1 to 59 and SEQ ID NO: 72 to 180.

In a particular embodiment, disclosed is a device comprising:

- an antibody which binds an epitope-antigen (peptide) comprising or consisting of a sequence selected from SEQ ID NO: 72 to 77, and
- at least one antibody which binds an epitope-antigen (peptide) comprising or consisting of a sequence selected from SEQ ID NO: 1 to 71 and SEQ ID NO: 78 to 180.

In a particular embodiment, disclosed is a device comprising:

- an antibody which binds an epitope-antigen (peptide) comprising or consisting of a sequence selected from SEQ ID NO: 78 to 82, and
- at least one antibody which binds an epitope-antigen (peptide) comprising or consisting of a sequence selected from SEQ ID NO: 1 to 77 and SEQ ID NO: 83 to 180.

In a particular embodiment, disclosed is a device comprising:

- an antibody which binds an epitope-antigen (peptide) comprising or consisting of a sequence selected from SEQ ID NO: 83 to 95, and
- at least one antibody which binds an epitope-antigen (peptide) comprising or consisting of a sequence selected from SEQ ID NO: 1 to 82 and SEQ ID NO: 96 to 180.

In a particular embodiment, disclosed is a device comprising:

- an antibody which binds an epitope-antigen (peptide) comprising or consisting of a sequence selected from SEQ ID NO: 96 to 108, and
- at least one antibody which binds an epitope-antigen (peptide) comprising or consisting of a sequence selected from SEQ ID NO: 1 to 95 and SEQ ID NO: 109 to 180.

In a particular embodiment, disclosed is a device comprising:

- an antibody which binds an epitope-antigen (peptide) comprising or consisting of a sequence selected from SEQ ID NO: 109 to 114, and
- at least one antibody which binds an epitope-antigen (peptide) comprising or consisting of a sequence selected from SEQ ID NO: 1 to 108 and SEQ ID NO: 115 to 180.

In a particular embodiment, disclosed is a device comprising:

- an antibody which binds an epitope-antigen (peptide) comprising or consisting of a sequence selected from SEQ ID NO: 115 to 126, and
- at least one antibody which binds an epitope-antigen (peptide) comprising or consisting of a sequence selected from SEQ ID NO: 1 to 114 and SEQ ID NO: 127 to 180.

In a particular embodiment, disclosed is a device comprising:

- an antibody which binds an epitope-antigen (peptide) comprising or consisting of a sequence selected from SEQ ID NO: 127 to 135, and
- at least one antibody which binds an epitope-antigen (peptide) comprising or consisting of a sequence selected from SEQ ID NO: 1 to 126 and SEQ ID NO: 136 to 180.

In a particular embodiment, disclosed is a device comprising:

- an antibody which binds an epitope-antigen (peptide) comprising or consisting of a sequence selected from SEQ ID NO: 136 to 141, and
- at least one antibody which binds an epitope-antigen (peptide) comprising or consisting of a sequence selected from SEQ ID NO: 1 to 135 and SEQ ID NO: 142 to 180.

In a particular embodiment, disclosed is a device comprising:

- an antibody which binds an epitope-antigen (peptide) comprising or consisting of a sequence selected from SEQ ID NO: 142 to 147, and
- at least one antibody which binds an epitope-antigen (peptide) comprising or consisting of a sequence selected from SEQ ID NO: 1 to 141 and SEQ ID NO: 148 to 180.

In a particular embodiment, disclosed is a device comprising:

- an antibody which binds an epitope-antigen (peptide) comprising or consisting of a sequence selected from SEQ ID NO: 148 to 154, and
- at least one antibody which binds an epitope-antigen (peptide) comprising or consisting of a sequence selected from SEQ ID NO: 1 to 147 and SEQ ID NO: 155 to 180.

In a particular embodiment, disclosed is a device comprising:

- an antibody which binds an epitope-antigen (peptide) comprising or consisting of a sequence selected from SEQ ID NO: 155 to 172, and
- at least one antibody which binds an epitope-antigen (peptide) comprising or consisting of a sequence selected from SEQ ID NO: 1 to 154 and SEQ ID NO: 173 to 180.

In a particular embodiment, disclosed is a device comprising:

- an antibody which binds an epitope-antigen (peptide) comprising or consisting of a sequence selected from SEQ ID NO: 173 to 180, and
- at least one antibody which binds an epitope-antigen (peptide) comprising or consisting of a sequence selected from SEQ ID NO: 1 to 172.

In a particular embodiment, the invention is a device comprising at least one antibody selected from:

- an antibody which binds an epitope-antigen (peptide) comprising or consisting of a sequence selected from SEQ ID NO: 1 to 5, and which also binds a human serum albumin,
- an antibody which binds an epitope-antigen (peptide) comprising or consisting of a sequence selected from SEQ ID NO: 6 to 13, and which also binds a human alpha-1B-glycoprotein,
- an antibody which binds an epitope-antigen (peptide) comprising or consisting of a sequence selected from SEQ ID NO: 14 to 16, and which also binds a human alpha-1-antichymotrypsin protein,
- an antibody which binds an epitope-antigen (peptide) comprising or consisting of a sequence selected from SEQ ID NO: 17 to 19, and which also binds a human alpha-1-antichymotrypsin protein,
- an antibody which binds an epitope-antigen (peptide) comprising or consisting of a sequence selected from SEQ ID NO: 20 to 26, and which also binds a human complement C3 protein,
- an antibody which binds an epitope-antigen (peptide) comprising or consisting of a sequence selected from SEQ ID NO: 27 to 33, and which also binds a human apolipoprotein B100 protein,
- an antibody which binds an epitope-antigen (peptide) comprising or consisting of a sequence selected from SEQ ID NO: 34 to 48, and which also binds a human alpha-1-antichymotrypsin protein,
- an antibody which binds an epitope-antigen (peptide) comprising or consisting of a sequence selected from SEQ ID NO: 49 to 59, and which also binds a human apolipoprotein B100 protein,
- an antibody which binds an epitope-antigen (peptide) comprising or consisting of a sequence selected from SEQ ID NO: 60 to 71, and which also binds a human complement C4Bp protein,
- an antibody which binds an epitope-antigen (peptide) comprising or consisting of a sequence selected from SEQ ID NO: 72 to 77, and which also binds a human complement C9 protein,
- an antibody which binds an epitope-antigen (peptide) comprising or consisting of a sequence selected from SEQ ID NO: 78 to 82, and which also binds a human complement C9 protein,
- an antibody which binds an epitope-antigen (peptide) comprising or consisting of a sequence selected from SEQ ID NO: 83 to 95, and which also binds a human complement C9 protein,
- an antibody which binds an epitope-antigen (peptide) comprising or consisting of a sequence selected from SEQ ID NO: 96 to 108, and which also binds a human complement C9 protein,
- an antibody which binds an epitope-antigen (peptide) comprising or consisting of a sequence selected from SEQ ID NO: 109 to 114, and which also binds a human alpha-2-HS-glycoprotein,
- an antibody which binds an epitope-antigen (peptide) comprising or consisting of a sequence selected from SEQ ID NO: 115 to 126, and which also binds a human complement C5 protein,
- an antibody which binds an epitope-antigen (peptide) comprising or consisting of a sequence selected from SEQ ID NO: 127 to 135, and which also binds a human apolipoprotein B100 protein,
- an antibody which binds an epitope-antigen (peptide) comprising or consisting of a sequence selected from SEQ ID NO: 136 to 141, and which also binds a human complement C4Bp protein,
- an antibody which binds an epitope-antigen (peptide) comprising or consisting of a sequence selected from SEQ ID NO: 142 to 147, and which also binds a human complement C4Bp protein,
- an antibody which binds an epitope-antigen (peptide) comprising or consisting of a sequence selected from SEQ ID NO: 148 to 154, and which also binds the human factor H protein,

- an antibody which binds an epitope-antigen (peptide) comprising or consisting of a sequence selected from SEQ ID NO: 155 to 172, and which also binds a human desmoplakin protein, and
- an antibody which binds an epitope-antigen (peptide) comprising or consisting of a sequence selected from SEQ ID NO: 173 to 180, and which also binds a human gelsolin protein.

In a particular embodiment, disclosed is a device comprising 1, 2, 3, 4, 5, 6, 7, 8, 9, 10, 11, 12, 13, 14, 15, 16, 17, 18, 19, 20, 21 or 22 antibodies selected from:

- an antibody which binds an epitope-antigen (peptide) comprising or consisting of a sequence selected from SEQ ID NO: 1 to 5, and which also binds a human serum albumin,
- an antibody which binds an epitope-antigen (peptide) comprising or consisting of a sequence selected from SEQ ID NO: 6 to 13, and which also binds a human alpha-1B-glycoprotein,
- an antibody which binds an epitope-antigen (peptide) comprising or consisting of a sequence selected from SEQ ID NO: 14 to 16, and which also binds a human alpha-1-antichymotrypsin protein,
- an antibody which binds an epitope-antigen (peptide) comprising or consisting of a sequence selected from SEQ ID NO: 17 to 19, and which also binds a human alpha-1-antichymotrypsin protein,
- an antibody which binds an epitope-antigen (peptide) comprising or consisting of a sequence selected from SEQ ID NO: 20 to 26, and which also binds a human complement C3 protein,
- an antibody which binds an epitope-antigen (peptide) comprising or consisting of a sequence selected from SEQ ID NO: 27 to 33, and which also binds a human apolipoprotein B100 protein,
- an antibody which binds an epitope-antigen (peptide) comprising or consisting of a sequence selected from SEQ ID NO: 34 to 48, and which also binds a human alpha-1-antichymotrypsin protein,
- an antibody which binds an epitope-antigen (peptide) comprising or consisting of a sequence selected from SEQ ID NO: 49 to 59, and which also binds a human apolipoprotein B100 protein,
- an antibody which binds an epitope-antigen (peptide) comprising or consisting of a sequence selected from SEQ ID NO: 60 to 71, and which also binds a human complement C4Bp protein,
- an antibody which binds an epitope-antigen (peptide) comprising or consisting of a sequence selected from SEQ ID NO: 72 to 77, and which also binds a human complement C9 protein,
- an antibody which binds an epitope-antigen (peptide) comprising or consisting of a sequence selected from SEQ ID NO: 78 to 82, and which also binds a human complement C9 protein,
- an antibody which binds an epitope-antigen (peptide) comprising or consisting of a sequence selected from SEQ ID NO: 83 to 95, and which also binds a human complement C9 protein,
- an antibody which binds an epitope-antigen (peptide) comprising or consisting of a sequence selected from SEQ ID NO: 96 to 108, and which also binds a human complement C9 protein,
- an antibody which binds an epitope-antigen (peptide) comprising or consisting of a sequence selected from SEQ ID NO: 109 to 114, and which also binds a human alpha-2-HS-glycoprotein,
- an antibody which binds an epitope-antigen (peptide) comprising or consisting of a sequence selected from SEQ ID NO: 115 to 126, and which also binds a human complement C5 protein,
- an antibody which binds an epitope-antigen (peptide) comprising or consisting of a sequence selected from SEQ ID NO: 127 to 135, and which also binds a human apolipoprotein B100 protein,
- an antibody which binds an epitope-antigen (peptide) comprising or consisting of a sequence selected from SEQ ID NO: 136 to 141, and which also binds a human complement C4Bp protein,
- an antibody which binds an epitope-antigen (peptide) comprising or consisting of a sequence selected from SEQ ID NO: 142 to 147, and which also binds a human complement C4Bp protein,
- an antibody which binds an epitope-antigen (peptide) comprising or consisting of a sequence selected from SEQ ID NO: 148 to 154, and which also binds the human factor H protein,
- an antibody which binds an epitope-antigen (peptide) comprising or consisting of a sequence selected from SEQ ID NO: 155 to 172, and which also binds a human desmoplakin protein, and
- an antibody which binds an epitope-antigen (peptide) comprising or consisting of a sequence selected from SEQ ID NO: 173 to 180, and which also binds a human gelsolin protein.

In a particular embodiment, the invention is a device comprising:

- an antibody which binds an epitope-antigen (peptide) comprising or consisting of a sequence selected from SEQ ID NO: 1 to 5, and which also binds a human serum albumin,
- an antibody which binds an epitope-antigen (peptide) comprising or consisting of a sequence selected from SEQ ID NO: 6 to 13, and which also binds a human alpha-1B-glycoprotein,

- an antibody which binds an epitope-antigen (peptide) comprising or consisting of a sequence selected from SEQ ID NO: 14 to 16, and which also binds a human alpha-1-antichymotrypsin protein,
- an antibody which binds an epitope-antigen (peptide) comprising or consisting of a sequence selected from SEQ ID NO: 17 to 19, and which also binds a human alpha-1-antichymotrypsin protein,
- an antibody which binds an epitope-antigen (peptide) comprising or consisting of a sequence selected from SEQ ID NO: 20 to 26, and which also binds a human complement C3 protein,
- an antibody which binds an epitope-antigen (peptide) comprising or consisting of a sequence selected from SEQ ID NO: 27 to 33, and which also binds a human apolipoprotein B100 protein,
- an antibody which binds an epitope-antigen (peptide) comprising or consisting of a sequence selected from SEQ ID NO: 34 to 48, and which also binds a human alpha-1-antichymotrypsin protein,
- an antibody which binds an epitope-antigen (peptide) comprising or consisting of a sequence selected from SEQ ID NO: 49 to 59, and which also binds a human apolipoprotein B100 protein,
- an antibody which binds an epitope-antigen (peptide) comprising or consisting of a sequence selected from SEQ ID NO: 60 to 71, and which also binds a human complement C4Bp protein,
- an antibody which binds an epitope-antigen (peptide) comprising or consisting of a sequence selected from SEQ ID NO: 72 to 77, and which also binds a human complement C9 protein,
- an antibody which binds an epitope-antigen (peptide) comprising or consisting of a sequence selected from SEQ ID NO: 78 to 82, and which also binds a human complement C9 protein,
- an antibody which binds an epitope-antigen (peptide) comprising or consisting of a sequence selected from SEQ ID NO: 83 to 95, and which also binds a human complement C9 protein,
- an antibody which binds an epitope-antigen (peptide) comprising or consisting of a sequence selected from SEQ ID NO: 96 to 108, and which also binds a human complement C9 protein,
- an antibody which binds an epitope-antigen (peptide) comprising or consisting of a sequence selected from SEQ ID NO: 109 to 114, and which also binds a human alpha-2-HS-glycoprotein,
- an antibody which binds an epitope-antigen (peptide) comprising or consisting of a sequence selected from SEQ ID NO: 115 to 126, and which also binds a human complement C5 protein,
- an antibody which binds an epitope-antigen (peptide) comprising or consisting of a sequence selected from SEQ ID NO: 127 to 135, and which also binds a human apolipoprotein B100 protein,
- an antibody which binds an epitope-antigen (peptide) comprising or consisting of a sequence selected from SEQ ID NO: 136 to 141, and which also binds a human complement C4Bp protein,
- an antibody which binds an epitope-antigen (peptide) comprising or consisting of a sequence selected from SEQ ID NO: 142 to 147, and which also binds a human complement C4Bp protein,
- an antibody which binds an epitope-antigen (peptide) comprising or consisting of a sequence selected from SEQ ID NO: 148 to 154, and which also binds the human factor H protein,
- an antibody which binds an epitope-antigen (peptide) comprising or consisting of a sequence selected from SEQ ID NO: 155 to 172, and which also binds a human desmoplakin protein, and
- an antibody which binds an epitope-antigen (peptide) comprising or consisting of a sequence selected from SEQ ID NO: 173 to 180, and which also binds a human gelsolin protein.

In a particular embodiment, the invention relates to a device comprising at least one antibody selected from the monoclonal antibodies: Bsi0097, Bsi0116, Bsi0142, Bsi0144, Bsi0186, Bsi0190, Bsi0214, Bsi0221, Bsi0300, Bsi0439, Bsi0581, Bsi0585, Bsi0639, Bsi0686, Bsi0759, Bsi0782, Bsi0789, Bsi1154, Bsi1186, Bsi1328, Bsi1517 and Bsi2487, or a fragment or derivative thereof having the same antigen specificity.

In a particular embodiment, the invention relates to a device comprising 1, 2, 3, 4, 5, 6, 7, 8, 9, 10, 11, 12, 13, 14, 15, 16, 17, 18, 19, 20, 21 or 22 antibodies selected from the monoclonal antibodies: Bsi0097, Bsi0116, Bsi0142, Bsi0144, Bsi0186, Bsi0190, Bsi0214, Bsi0221, Bsi0300, Bsi0439, Bsi0581, Bsi0585, Bsi0639, Bsi0686, Bsi0759, Bsi0782, Bsi0789, Bsi1154, Bsi1186, Bsi1328, Bsi1517 and Bsi2487.

In a particular embodiment, the invention relates to a device comprising: Bsi0097, Bsi0116, Bsi0142, Bsi0144, Bsi0186, Bsi0190, Bsi0214, Bsi0221, Bsi0300, Bsi0439, Bsi0581, Bsi0585, Bsi0639, Bsi0686, Bsi0759, Bsi0782, Bsi0789, Bsi1154, Bsi1186, Bsi1328, Bsi1517 and Bsi2487.

In a particular embodiment, disclosed is a device as defined above wherein said at least one antibody, or fragment or derivative thereof, is immobilized on a support.

The support may be a membrane, a slide, a microarray, a chip or micro-bead based detection system.

A device of the invention can be used for *in vitro* or *ex vivo* detection / diagnosis / prognosis of lung cancer in a subject.

In particular, a device of the invention can be used in a competitive assay, a capture assay or an sandwich ELISA assay, preferably a competitive assay.

A device of the invention can be used for diagnosing, providing prognosis, monitoring, characterizing, determining a severity of, confirming a presence of, or confirming an absence of LC in a subject, the device comprising:

a) an input module for receiving as an input levels of one or more epitope-antigen;

(b) a processor configured to: perform an algorithm, based on logistic regression models with forward or enter method, adjusting the levels of each of the one or more epitope-antigen to a corresponding control value, thereby providing one or more epitope-antigen levels; perform a real function algorithm manipulating the one or more epitope-antigen levels by multiplying one or more variables by one or more corresponding weight factors, wherein a level of each of the one or more adjusted epitope-antigen levels is input into a specific variable of the one or more variables, wherein the corresponding weight factor is unique for each specific variable, wherein at least one of the corresponding weight factors is different from one; and

(c) an output module for outputting an index value, wherein the index value indicates diagnosis, prognosis, characterization, a monitored aspect, determination of the severity, confirmation of the presence, or confirmation of the absence, of LC in the subject, wherein the device comprises at least one antibody which binds an epitope-antigen comprising or consisting of a sequence selected from SEQ ID NO: 1 to 180, or one fragment or derivative of such an antibody having the same antigen specificity.

A further device of the invention can be used for diagnosing, providing prognosis, monitoring, characterizing, determining a severity of, confirming a presence of, or confirming an absence of LC in a subject, the device comprising:

a) an input module for receiving as an input levels of one or more epitope-antigen;

(b) a processor configured to: perform an algorithm, based on logistic regression models with forward or enter method, adjusting the levels of each of the one or more epitope-antigen to a corresponding control value, thereby providing one or more epitope-antigen levels; perform a real function algorithm manipulating the one or more epitope-antigen levels by multiplying one or more variables by one or more corresponding weight factors, wherein a level of each of the one or more adjusted epitope-antigen levels is input into a specific variable of the one or more variables, wherein the corresponding weight factor is unique for each specific variable, wherein at least one of the corresponding weight factors is different from one; and

(c) an output module for outputting an index value, wherein the index value indicates diagnosis, prognosis, characterization, a monitored aspect, determination of the severity, confirmation of the presence, or confirmation of the absence, of LC in the subject, wherein the device comprises at least five (5) antibodies wherein

- the first antibody specifically binds at least one epitope-antigen comprising or consisting of the sequences selected from the group consisting of SEQ ID NO: 14 to 16,
- the second antibody specifically binds at least one epitope-antigen comprising or consisting of the sequences selected from the group consisting of SEQ ID NO: 34 to 48,
- the third antibody specifically binds at least one epitope-antigen comprising or consisting of the sequences selected from the group consisting of SEQ ID NO: 72 to 77,
- the fourth antibody specifically binds at least one epitope-antigen comprising or consisting of the sequences selected from the group consisting of SEQ ID NO: 109 to 114, and
- the fifth antibody specifically binds at least one epitope-antigen comprising or consisting of the sequences selected from the group consisting of SEQ ID NO: 142 to 147,

or one fragment or derivative of such antibodies having the same antigen specificity.

The embodiments of the method of *in vitro* or *ex vivo* detection / diagnosis / prognosis of lung cancer with the antibodies as defined above apply mutatis mutandis to a method of *in vitro* or *ex vivo* detection / diagnosis / prognosis of lung cancer with the antibodies of the invention.

A further object of the disclosure (not claimed) is a kit comprising a device as defined above and a reagent to perform or detect (or quantify) an immune reaction, particularly an antibody-antigen complex. Reagents include labels, buffers, substrates, etc. The kits typically comprise containers for the different reagents and products, and may further comprise a support or other device suitable to perform the assay.

The antibodies can be used individually or in combination to measure the level of the cognate epitope-antigen (analyte) in bio-fluids including serum, plasma, urine, cerebrospinal fluid, bronchoalveolar lavage (BAL) fluid, sputum, tear, sweat, amniotic fluid and inflammatory exudate, feces, using any number of detection technologies or platforms such as, without limitation Capture assay, Sandwich assay, Competition assay, Radio-immuno-assays, Enzyme labels with substrates that generate colored, fluorescent, chemiluminescent, or electrochemically-active products, Fluorescence, fluorescent polarization, Chemiluminescence, Optical and colorimetric, Electrochemiluminescence, Time-resolved fluorescence, Surface plasmon resonance, Evanescent wave, Multiwell plate (ELISA), Individual assay, Multiplex assay, Latex bead - multiplex assay, Microarray (Laminar surface) - multiplex assay, Glass, Ceramic (like Randox), Plate based assays, Strip based assays, dipsticks, Closed systems immunoassays.

Preferred assay format include:

**Capture inhibition assay**

A competitive Elisa assay, also named Capture inhibition assay (CIA) is used in the present application. The binding of a labeled tracer protein by a single antibody is inhibited by preincubation of a bio-fluid to indirectly quantify the analyte.

The antibody is immobilized directly to the support or captured by an affinity reagent such as an anti-mouse IgG antibody coated onto the support. The immobilized antibody is then incubated with any of the above mentioned body fluids. The immobilized antibody/antigen complex is then incubated with a labeled tracer consisting of either (i) any of the above mentioned body fluids in which the proteins have been labeled with a detection molecule such as biotin, with or without pre-treatment to remove abundant proteins, or (ii) a purified or recombinant protein recognized (bound) by the monoclonal antibody, or (iii) an epitope-antigen (peptide) which is recognized (bound) by the monoclonal antibody. The labeled protein or epitope-antigen (peptide) which is bound by the antibody is detected by the addition of an appropriate detection reagent which binds to the label such as avidin or streptavidin which has been modified to be compatible with one of the detection technologies described in the section "detection technology."

The level of the specific protein in the unlabeled bio-fluid is determined as a function of the inhibition of signal.

**Capture assay**

An assay carried out using a single immobilized antibody (multiwall plate, latex bead, microarray, etc.) which captures a specific labeled protein from a bio-fluid, the detection which is measured using appropriate detection reagents as detailed in the following paragraph.

The antibody is immobilized directly to the support or captured by an affinity reagent such as an anti-mouse IgG antibody coated onto the support. The immobilized antibody is then incubated with any of the above mentioned body fluids in which the proteins have been labeled with a detection molecule such as biotin, with or without pre -treatment to remove abundant proteins. The labeled protein which is bound by the antibody is detected by the addition of an appropriate detection reagent which binds to the label such as avidin or streptavidin which has been modified to be compatible with one of the detection technologies described in the section "detection technology."

The resulting signal provides a quantitative measure of the amount of labeled protein bound by the antibody.

**Sandwich ELISA**

An assay using two antibodies, the first which is immobilized on a support (multiwell plate, latex bead, microarray, etc.) which binds a specific epitope of a specific protein from a biofluid, the detection which is measured using a labeled second antibody against different epitope(s) of the same protein and appropriate detection reagents as detailed in the following paragraph.

The first antibody is immobilized directly to the support or captured by an affinity reagent such as an anti-mouse IgG antibody coated onto the support. The immobilized antibody is then incubated with any of the above mentioned body fluids, with or without pre-treatment to remove abundant proteins. The antibody/antigen complex is then incubated with a second antibody, made against the same protein, such as a polyclonal antibody which has been labeled with a detection molecule such as biotin. The bound antibody is detected by the addition of an appropriate detection reagent which binds to the label such as avidin or streptavidin which has been modified to be compatible with one of the detection technologies described in the section "detection technology".

The resulting signal provides a quantitative measure of the amount of protein bound by the antibody.

Preferred detection technologies include:

**Enzyme labels with substrates that generate colored, fluorescent, chemiluminescent, or electrochemically-active products**

The detection reagent (for example streptavidin or avidin, which binds to biotin) is coupled to an enzyme such as horseradish peroxidase which is capable of catalyzing:

- an appropriate colorimetric substrate of which the product demonstrates maximal absorbance at a given wavelength allowing the quantitative measurement of the labeled protein by determining the optical density of the final product in the well at or near the wavelength of maximal absorbance.
- a chemiluminescent substrate to a sensitized reagent which upon oxidation emits light, providing the quantitative measurement of the labeled protein.

- a chemiluminescent substrate to a sensitized reagent which upon the application of an electrical current emits light, providing the quantitative measurement of the labeled protein.

**Fluorescence**

The detection reagent (for example streptavidin or avidin, which binds to biotin) is coupled to a fluorescent tag.
Preferred platform Technologies include:

**Multiwell Plate**

◦ Single test: one antibody is immobilized per well either directly or indirectly using a capture reagent such as goat anti-mouse antibody.
◦ Multiplex: 2 or more antibodies are immobilized in a single well by deposition in a pattern.

**Latex bead**

Two or more antibodies are immobilized onto a latex bead between x and y micron.

**Arrays, microarrays, and nanoarrays**

Two or more antibodies are spotted onto an activated laminar surface with a spot diameter between 100 $\mu$m - 5 mm (arrays), 2 $\mu$m - 100 $\mu$m (microarrays), 10 nm-2 $\mu$m (nano-arrays) The surface can be composed of glass, plastic, ceramic, carbon nanotube lattice etc.
The method can be performed at any stage of the disease, such as early or late stage, to confirm or reject a prior diagnosis, select patients for surgery, classify cancer type or severity, or monitor patients. The test may also be conducted before disease symptoms, as a first line detection. Clinical diagnostic application of a lung cancer plasma (serum) test will be useful e.g., for patients who are suspected to have lung cancer because of a suspicious nodule that has been detected by imaging of the lung (CT scan). Nodules < 0.5 cm are suspicious and quite frequent in the populations. Nodules > 0.5 cm, but < 1.1 cm represent an increased likelihood of being cancerous, however challenging to find by surgery and invasive endoscopic procedures. It is important to select patients from this group who's nodule are definitively cancerous to reduce the burden of futile surgeries and other invasive diagnostic procedures.
The test will avoid futile thoracotomies and unnecessary and expensive imaging technologies that are not specific enough and expose the patients to potentially harmful irradiation, and missed cures as observed patients could receive the test repeatedly.

**PEPTIDES**

A further object of the disclosure (not claimed) is an epitope-antigen (peptide) consisting of an amino acid sequence selected from SEQ ID NO: 1 to 180.
The disclosure also relates to the use of an epitope-antigen (peptide) consisting of an amino acid sequence selected from SEQ ID NO: 1 to 180 for the *in vitro* or *ex vivo* detection / diagnosis / prognosis of lung cancer.
Further aspects and advantages of the invention will be disclosed in the following examples, which should be considered illustrative.

**FIGURES**

Figure 1: shows the analytical evaluation of the QP69 kit - inter-assay reproducibility (A), Analytical evaluation of the Q.P69 kit - lot-to-lot variation (B).

Figure 2: shows the results concerning epitopes of individual plasma proteins behave differently with respect to association with LC. Three epitopes, BSI0581, BSI0449 and BSI0639 of C9, box plot of relative representation RLU/RLUmax values (A). Multiple epitopes of C9, C4BP, alpha2-HS-Glycoprotein and CFH expressed via ROC display (B).

**Figure** 3:
**A:** shows the results concerning the LC21 model of lung cancer discriminating plasma protein epitopes. The study was done with the QPLC21 biochip run on the Evidence Investigator platform in CIA. Cohorts included the entire BD cohort and selected set of NKTH cohort of symptomatic lung cancer patients and controls. **B:** Epitopes included in the LC21 model. **C:** Algorithm of the LC model, which is derived from logistic regression analysis by Enter method. **D:** Table describes the ROC and specificity and sensitivity values at various cut-offs of the ROC curve. **E:** composition of the cohorts.

**Figure 4:**
**A:** shows the results concerning the Extended M61 model of lung cancer discriminating plasma protein epitopes. The study was done with the QPLC21 biochip run on the Evidence Investigator platform in CIA. Cohorts included the audited BD samples with matched controls (age, sex, BMI, smoking habit, COPD status). **B:** Epitopes included in the Extended M61 model. **C:** Algorithm of the LC model, which is derived from logistic regression analysis, by Enter method, of manually selected BSI variables of NSCLC/CE subpopulation of mixed tracer dataset **D:** Table describes the ROC and specificity and sensitivity values at various cut-offs of the ROC curve. **E:** composition of the cohorts.

**Figure 5:**
**A:** shows the results concerning the Extended M63 model of lung cancer discriminating plasma protein epitopes. The study was done with the QPLC21 biochip run on the Evidence Investigator platform in CIA. Cohorts included the audited BD samples with matched controls (age, sex, BMI, smoking habit, COPD status). **B:** Epitopes included in the Extended M63 model. **C:** Algorithm of the Extended M63, which is derived from logistic regression analysis, by ENTER method of manually selected BSI variables of NSCLC/CE subpopulation of mixed and depleted tracer dataset. **D:** Table describes the ROC and specificity and sensitivity values at various cut-offs of the ROC curve. **E:** composition of the cohorts.

**Figure 6:**
**A:** shows the results concerning the epitopes included in the M48 model. **B:** M48 model was developed by Forward method using preselected variables based on Mann-Whitney U test / independent t-test on QM504-QP507 NSCLC dataset. C: The performance of M48 on the audited BD cohort (ALL) and the early (CE) and late subsections (CL). The study was done with the QPLC21 biochip run on the Evidence Investigator platform in CIA. Tables describes the ROC and specificity and sensitivity values at various cut-offs of the ROC curve.

**Figure 7:**
**A:** shows the results concerning the 96wQPLC6 M13 model of lung cancer discriminating plasma protein epitopes. The study was done with the QPLC6 mAbs on 96w ELISA platform in CIA. Cohorts included the audited BD late stage (LC stage III/B & IV) lung cancer patient samples with controls having CYFRA measurement as well. **B:** Epitopes included in the 96wQPLC6 M13 model. **C:** Algorithm of the 96wQPLC6 M13, which is derived from logistic regression analysis, by FORWARD method of BSI0144, BSI0214, BSI0221, BSI0581, BSI0759, BSI1186, ALBUMIN, CA125, CEA, CYFRA, TPAM, CRP, HE4 variables. **D:** Table describes the ROC and specificity and sensitivity values at various cut-offs of the ROC curve. **E:** composition of the cohort. **F:** Boxplot shows 96wQPLC6 M13 model prediction data by lung cancer stage.

**Figure 8:**
**A:** shows the results concerning the 96wQPLC6 M15 model of lung cancer discriminating plasma protein epitopes. The study was done with the QPLC6 mAbs on 96w ELISA platform in CIA. Cohort included the audited BD early stage (LC stage I & II) lung cancer patient samples with controls having CYFRA measurement as well. **B:** Epitopes included in the 96wQPLC6 M15 model. **C:** Algorithm of the 96wQPLC6 M15, which is derived from logistic regression analysis, by FORWARD method of BSI0144, BSI0214, BSI0221, BSI0581, BSI0759, BSI1186, CYFRA variables. **D:** Table describes the ROC and specificity and sensitivity values at various cut-offs of the ROC curve. **E:** composition of the cohort. **F:** Boxplot of 96wQPLC6 M15 model prediction data by lung cancer stage.

**Figure 9:**
**A:** shows the results concerning the 96wQPLC6 M9 model of lung cancer discriminating plasma protein epitopes. The study was done with the QPLC6 mAbs on 96w ELISA platform in CIA. Cohort included the audited BD early stage (LC stage I, II & III/A) lung cancer patient samples with controls having CYFRA measurement as well. **B:** Epitopes included in the 96wQPLC6 M9 model. **C:** Algorithm of the 96wQPLC6 M9, which is derived from logistic regression analysis, by ENTER method of BSI0144, BSI0214, BSI0221, BSI0581, BSI0759, BSI1186, CYFRA variables. **D:** Table describes the ROC and specificity and sensitivity values at various cut-offs of the ROC curve. **E:** composition of the cohort. **F:** Boxplot of 96wQPLC6 M9 model prediction data by lung cancer stage.

**Figure 10:** shows the results concerning the predicted performance comparison of Extended M61, M48 models with Extended M63, Model "A" is used to describe LDCT comparisons, while Model "B" refers to Chest-X ray related comparisons. Performance indices are given in %.

The clinical validation of the epitope profiling was done using biobanked plasma samples from multiple independent cross sectional cohorts of apparently healthy and cancer free individuals and from symptomatic lung cancer patients. In some of the cohorts, in order to reduce confounding effects control and cancer subjects were matched with respect to age, sex, COPD status, BMI and smoking habits.

Surprisingly the epitome profiling revealed epitopes of individual plasma proteins that behave variably with respect to association with lung cancer, a particular example is complement factor 9 (C9) epitope BSI0581which shows decreased representation in LC patients compared to control, while BSI0449 does not change and BSI0639 which shows increased levels (Figure 2A). More examples of epitope abundancy variabilities are shown for further epitopes of C9, epitopes of complement factor 4 binding protein (C4BP), alpha-2 HS-glycoprotein and complement factor-H (CFH) as visualized via receiver operator curve (ROC) display derived from epitope profiling of plasma samples from the NKTH cohort of 46 controls and 46 symptomatic lung cancer patients (Figure 2B). Individual epitopes are defined by mimotope peptide sequences Table 1.

The datasets were supplemented with cancer biomarker data (CA125, CEA, CYFRA, TPAM and HE4) and a few laboratory-tests (CRP and Albumin), then analyzed with descriptive and machine learning statistical tools. The most valuable epitopes and other variables associated with LC were prioritized based on the following criteria: (i) individual discriminatory power in descriptive statistical tests (ii) contribution to multivariate panels with strong discriminatory power.

These studies resulted in the selection of 22 epitopes that satisfied selection criteria (shown on Table 1.), where cognate protein ID and mimotope sequences (8,9) are also shown.

Next, in the validation phase new cycles of profiling was done with the selected 22 epitopes using audited biobanks (BD and NKTH cohorts of symptomatic lung cancer patients and controls) with matched criteria (age, sex, COPD status, BMI and smoking habit), the dataset was supplemented with cancer biomarker data (CA125, CEA, CYFRA, TPAM and HE4) and a few laboratory tests (CRP and Albumin), then analyzed with descriptive and machine learning statistical tools. Statistical model building was aimed at (i) obtaining powerful discriminatory power for all stages of LC (ii) obtaining powerful discriminatory power for early (Stage I-II and +/- IIIA) LC. (iii) Obtaining discriminatory power in the presence and absence of COPD.

Profiling epitopes on the Randox evidence investigator platform resulted in an algorithm termed LC21 derived from logistic regression analysis using the "Enter" methodology, data of 21 epitopes (Bsi0097, Bsi0116, Bsi0142, Bsi0144, Bsi0186, Bsi0190, Bsi0221, Bsi0439, Bsi0581, Bsi0585, Bsi0639, Bsi0686, Bsi0759, Bsi0789, Bsi2487, Bsi1154, Bsi1186, Bsi1517, Bsi1328, Bsi0300, Bsi0782) but no other variables provided accuracy of 0.842 (AUC of ROC) on the large cohort (BD and NKTH cohorts of symptomatic lung cancer patients and controls) (Figure 3 panels A-E), sensitivity was 58.4% at 90% specificity (Figure 3 panel D). To select fewer epitopes, tumor marker and laboratory test variable logistic regression analysis was done. Extended M61 model (Figure 4, panels A-E) was developed by Enter method on NSCLC/CE QM504 dataset, which also incorporates 9 manually selected variables (6 Bsi mAb + 2 TM + Alb). RLU/RLUmax% values of Bsi0144 (ACT), Bsi0581 (C9), Bsi0186 (ACT), Bsi2487 (Gelsolin), Bsi0190 (C3), Bsi0782 (C5), which tested with mixed tracer, plus CYFRA, CRP and Albumin levels used in the statistical computing based on the formula, which provided the best accuracy for early lung cancer (ROC AUC) of 0.761 on a cohort of all types of early LC, while 0.767 on non-small cell lung cancer (NSCLC), in this context early lung cancer group included stages I-IIIA where surgical intervention is possible.

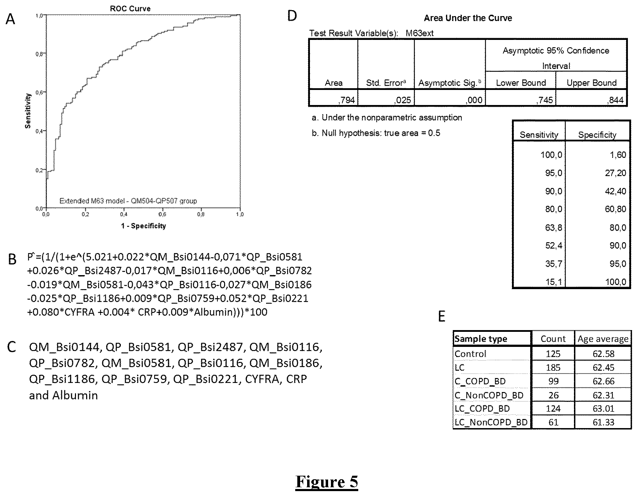

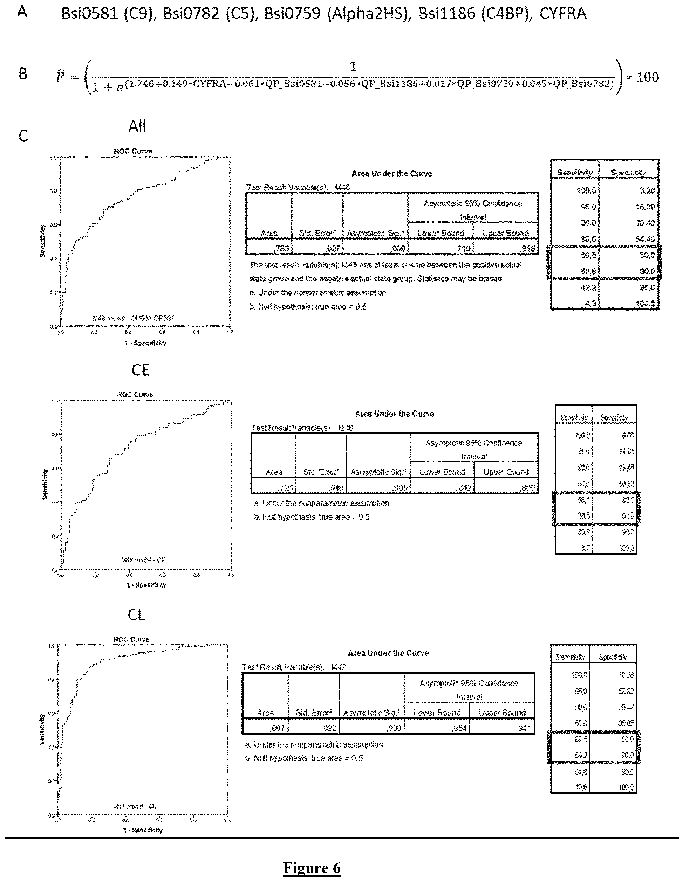

Profiling on the Randox Evidence Investigator platform resulted in another excellent example that includes the extended M63 model (Figure 5, A-E) which showed high level of accuracy on late stage cancer independent of cancer type (all and NSCLC only). As profiling many variables increases cost, we further searched for algorithms that incorporate minimal number of variables without major drop in the accuracy. An example is Model 48 (M48) which includes epitope variables BSI0581, BSI1186, BSI0759, BSI0782 and CYFRA (Figure 6, A-E). The epitope variables performed better than tumor biomarkers, on early LC samples with AUC: 0.712 compared to AUC: 0.679 (TM + Alb.), while on NSCLC-CE samples with AUC: 0.717 and AUC: 0.678 respectively. These differences were statistically significant. Algorithms Extended 63, Extended 61 and M48 showed results within 95% confidence when validated on independent cohorts or in cross validation testing.

**Meaning of the abbreviations in table E of figure 5:**

Control: control samples from BD cohort.

LC: lung cancer patients from BD cohort.

C_COPD_BD: subset of control samples from BD cohort having chronic obstructive pulmonary disease (COPD).

C_NonCOPD_BD: subset of control samples from BD cohort not having chronic obstructive pulmonary disease (COPD).

LC_COPD_BD: subset of lung cancer patients from BD cohort having chronic obstructive pulmonary disease (COPD).

LC_NonCOPD_BD: subset of lung cancer patients from BD cohort not having chronic obstructive pulmonary disease (COPD).

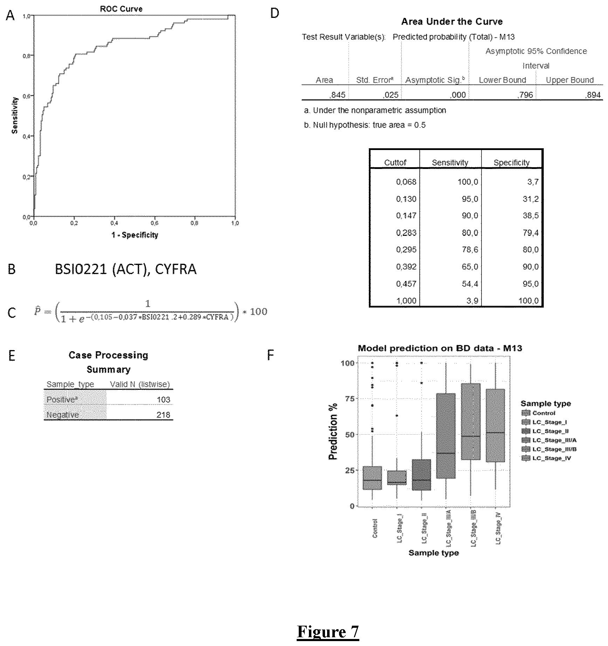

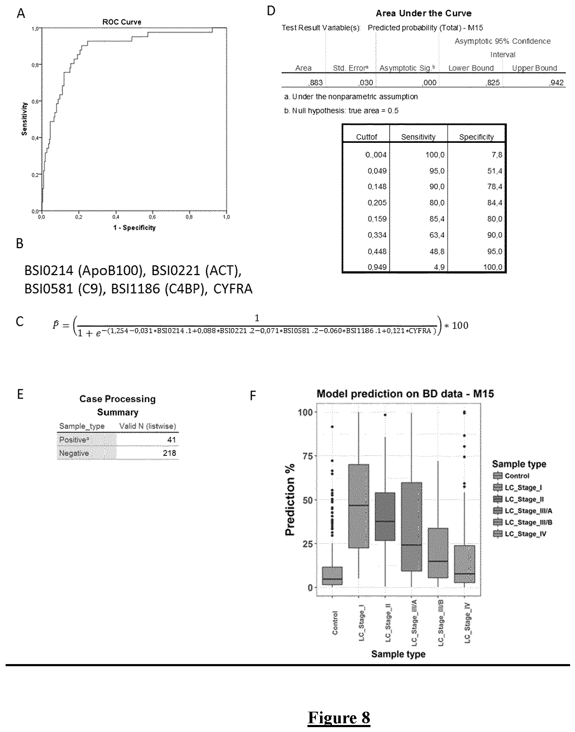

In order to demonstrate that epitope profiling works on classical 96 well microplate platform in ELISA setting we show examples of the epitomic panel 96wQPLC6 M13 (Figure 7, A-F) for the detection of late stage LC (stages III-IV), the 96wQPLC6 M15 (Figure 8, A-F) panel for the detection of early stage LC (stages I-II) and the panel 96wQPLC M9 (Figure 6, A-F) for the detection of all LC stages in the presence or absence of COPD. It is of particular interest that panel M15 presented very high accuracy for early stage lung cancer and that its discrimination power declined for late stage (III-IV) cancer (Figure 8, F), while model M13 showed inverse results (Figure 7, F).

## EXAMPLES

**Clinical samples:**

Two clinical sample cohorts were collected:

**NKTH cohort:** [IRB permits respectively: 3049-2009, 3140-2010 were from the Regional and Institutional Ethics Committee, Medical and Health Science Center, University of Debrecen (DE OEC RKEB/IKEB)]. This cohort contained 153 lung cancer, 107 colon cancer, 106 breast cancer, 152 prostate cancer, 26 ovarian cancer patients and 500 controls were enrolled in this study (320 healthy individuals, 119 individuals with diabetes mellitus and 61 with benign prostate hyperplasia) but only the healthy individuals served as controls for the LC patients. The inclusion criteria were strict for the healthy controls as individuals without potentially confounding accompanying diseases were involved. In the NKTH biobank the patients were matched with respect to age and sex.

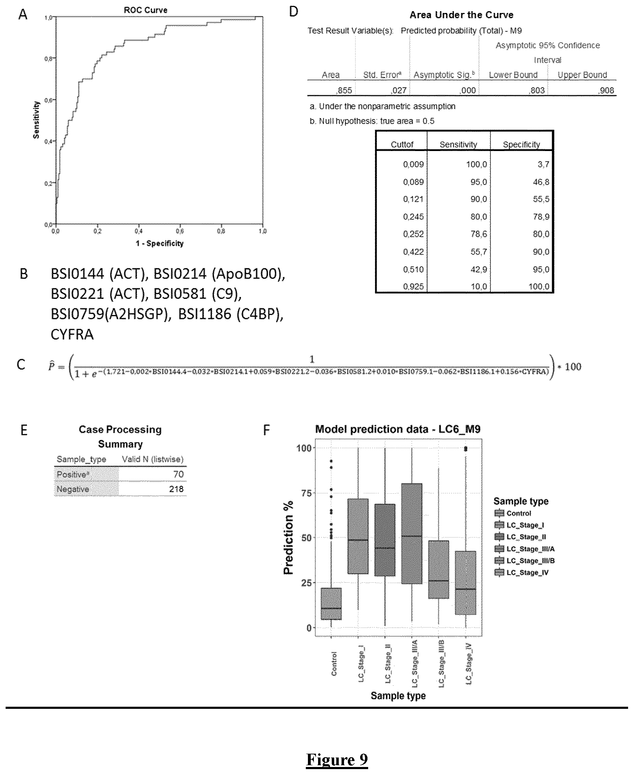

**BioDiagnostica (BD):** project was conducted under the permit: 11739/2014/EKU (107/2014 and in amended form 417/2014 from the Medical Research Council of Hungary (ETT TUKEB) on a multicentre cohort of 908 samples, 425 LC and 483 controls collected from four lung cancer treatment centers of Hungary (Semmelweis Hospital, Miskolc; Departments of Pulmonology of the University of Debrecen; University of Szeged and Semmelweis Medical University, Budapest). In this project control subjects with COPD were collected (because the majority of LC patients have COPD) and sub-cohorts - in addition to matched sex and BMI - were also matched with respect to COPD state, smoking habit and ethnicity in order to reduce the confounding impacts. For the lung cancer studies described on Fig 2B-C, Fig 6-9, samples were picked exclusively from the BD collection. Samples having routing cancer biomarker data (TM) data were matched pairwise by the following criteria to obtain control/early LC and control/late LC sub cohorts.

- Age: Maximal difference within pairs was ± 2 -5 yrs. (majority ± 2)
- Gender: Perfect match only.
- COPD status: sample COPD status classification was based on GOLD 2012 criteria. Matching: non COPD and COPD status A or B samples were matched (majority - 74 % - were complete match). In case of advanced COPD, C or D, samples could be matched only between each other.
- Smoking habit: Classification of samples was done in five categories based on the degree of smoking (pack/year). Beside non-smokers, there were four groups (A-D). Beside the complete match, matching A with B or B with C was also allowed.
- BMI: Donors were classified into 3 groups (A, B, C). Beside the complete match, matching A with B or B with C was also allowed.
- Ethnicity: There are Roma and White Caucasian samples. The few Roma LC samples were matched to Roma controls.
- Stage: Samples were divided into early and late stage samples. Early stage samples include: I.A, I.B, II.A, II.B, and III.A samples (operable cancer). Late samples: III.B, IV.
- Histology: Within the picked samples NSCLC subgroup of lung cancer samples were also classified.

In total 310 samples, (125 controls and 185 lung cancer with 43% Stage IV), were selected using the above listed criteria. There were 60 triplets, where the control sample had both early and late stage matched pairs. Beside these, there were 21 control/early LC and 44 control/late LC samples pairs. There were plasma samples in the case of which we had no 3 unthawed aliquots for the replicate measurements. In these cases one unthawed or thawed only "once" vial of plasma was aliquoted to obtain the sufficient number of vials for the experiments.

**QuantiPlasma monoclonal antibody library:**

mAb library produced as it was outlined in the Experimental protocols of previous publication[15,19]. Library members of QP69 & QP300 were selected based on their performance in capture ELISA assay with total plasma tracer or depleted plasma tracer respectively.

**Cognate protein antigen identification:**

Antigen identification was performed with immune affinity purification of cognate antigen of mAbs, followed by mass spectrometry analysis of eluted protein(s)[15].

**Epitope/mimotope identification:**

Ph.D.™-12 Phage Display Peptide Library Kit (New England Biolabs, Ipswich, MA) was used to identify mimotope peptide sequence(s) as it was outlined in details previously[20]. The obtained 12-mer peptide sequences of each screened mAbs were ranked first and then unique sequence(s) or motif(s) were determined. Unique sequence(s) of each antibody used to determine the epitope level redundancy, based on the frequencies of common mimetope peptide(s) of compared mAb pairs (see formula and description below)

$$RedXY = [(Xn1 * Ym1)*100] +... + [(Xni * Ymi)*100],$$

Xn: Frequency of X mAb's nth peptide: count of unique peptide n divided by all peptide count of mAb X.
Ym: Frequency of Y mAb's mth peptide: count of unique peptide m divided by all peptide count of mAb Y.
Xn1: Frequency of X mAb's first common peptide between X & Y mAbs,
Ym1: Frequency of Y mAb's first common peptide between X & Y mAbs,
Xni: Frequency of X mAb's ith common peptide between X & Y mAbs,
Ymi: Frequency of Y mAb's ith common peptide between X & Y mAbs.

**QuantiPlasma biochip experiments:**

BSI's QuantiPlasma monoclonal antibody library containing QP69 and QP300 and QPLC21 biochips array kits were produced by Randox Ltd. (Belfast, UK), employing its proprietary technology (13). QP69 mAbs spotted on three biochip arrays, while QP300 mAbs on 18 biochip arrays due to the limited number of testing regions on a 9X9 mm biochip surface. For each 18 sample measurement position (6 biochip racks handled at a time), two positions were used to measure maximal signal intensities for quality control and normalization purposes in case of QP69 biochip measurement. For QP300, 6 biochip racks provide only 3 measurement position, therefore one position used for maximal signal intensities and two positions for sample measurements. During the QPLC21 biochips tests, 1 out of 9 biochip within the biochip rack was used for the maximal signal intensity measurement. In total eight different QPLC21 biochip batches were used for the experiments (6800, 6801, 6802, 12773RD, 12774RD, 12775RD, 12776RD, and 12777RD). Biochips were processed according to the supplied protocol. Briefly, biotin labelled tracer was reconstituted with 4 mL deionized water and incubated for 30 minutes at room temperature on orbital shaker. Six biochip racks (nine biochips assembled together with a handle) inserted into the handling tray, then to each biochip 200 μL assay diluent buffer (19 mM Tris buffered saline pH 8 containing a protein matrix, surfactant and preservatives), 50 μL diluted sample and 50 μL reconstituted tracer were added. After 1 hour incubation in a Randox thermoshaker at 37 °C and 370 RPM of the biochips, the wash solution was discarded and biochips were washed 6 times with 350 μL washing buffer/biochip well (20 mM Tris buffered saline pH 7.2 containing surfactant and preservatives) followed 6 times rinsing for 2 minutes with 350 μL washing buffer/biochip well by gentle taping all edges of the handling tray for 10-15 seconds then let them soak. After the last washing cycle, residuals of wash buffer removed by taping the handling tray with biochip racks upside down onto the lint free tissue paper. Then biochips were incubated with 300 μL conjugate buffer (19 mM Tris buffered saline pH 7.2 containing a protein matrix, surfactant, preservatives and assay specific reagents labelled with horseradish peroxidase) for 1 hour in a Randox thermoshaker at 37 °C and 370 RPM then the previously described washing step were repeated. To the washed biochips 350 μL washing buffer was added to avoid drying the chip surface. Imaging was performed within 30 minutes after the last washing step by incubating one biochip rack of biochips at a time with 250 µL signal reagent mixture for 2 minutes and afterward transferring it into the Randox Evidence Investigator.

**ELISA description**

96 well plates (Corning 96 well plates, half-area clear polystyrene, high-binding, non-sterile, Sigma) were coated with 30 µL / well of 5 µg/ml goat anti-mouse IgG Human ads-UNLB (Southern Biotech, 1030-01) (GAM) in carbonate buffer (pH=9.6) at 37 °C for 60 minutes. After washing the plates for 2 times with PBS-Tween buffer, wells were blocked at 37 °C for overnight in 60 µl / well of Blocking buffer. Next day, followed 2 times washing coated plates add 30 µL of appropriately diluted mAb to each well from A1 to H7 positions (See the mAb distribution scheme and mAb dilutions below) of the microtiter plate, cover with aluminum foil and incubate the plate at 37°C for 1 hour.

mAb distribution scheme:

| | 1 | 2 | 3 | 4 | 5 | 6 | 7 | 8 | 9 | 10 | 11 | 12 |
|---|---|---|---|---|---|---|---|---|---|---|---|---|
| A | | | | | | | | | | | | |
| B | | | | | | | | | | | | |
| C | | | | | | | | | | | | |
| D | | | | | BSI mAb | | | | | | | |
| E | | | | | | | | | | | | |
| F | | | | | | | | | | | | |
| G | | | | | | | | | | | | |
| H | | | BSI mAb | | | | | | NO BSI mAb | | | |

| mAb name | Working Concentration | Diluent |
|---|---|---|
| BSI0144 | 0.1 µg/ml | Blocking buffer |
| BSI0214 | 0.3 µg/ml | Blocking buffer |
| BSI0221 | 0.1 µg/ml | Blocking buffer |
| BSI0581 | 0.1 µg/ml | Blocking buffer |
| BSI0759 | 0.3 µg/ml | Blocking buffer |
| BSI1186 | 0.3 µg/ml | Blocking buffer |

During the incubation period the following dilutions (see table 4 below), which also containing 3 µg/ml total plasma tracer (QPTR001) and 3 µg/ml HU14 depleted plasma tracer (QPTR003), were prepared in Blocking buffer from each plasma samples to be tested and from the positive and negative control pools.

Table 4

| mAb name | working dilution of plasma sample |
|---|---|
| BSI0144 | 3000x |
| BSI0214 | 10000x |
| BSI0221 | 1000x |
| BSI0581 | 1000x |
| BSI0759 | 1000x |
| BSI1186 | 300x |

To measure the maximal signal intensity and background (positive and negative technical control replicates) on each plate 3 µg/ml total plasma tracer (QPTR001) and 3 µg/ml HU14 depleted plasma tracer (QPTR003) was also prepared in Blocking buffer (1X Tracer mix). After 1 hour remove the BSI mAb solution and wash plates 3 times with 200 µ! PBS-TWEEN. 30 µL of the appropriate sample dilutions were distributed to the proper wells designated with "Sample-1" to "Sample-42" of the microtiter plate according to the following scheme.

| | Sample and controls distribution scheme: | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | Sample-1 | | Sample-9 | | Sample-17 | | Sample-22 | | Sample-29 | | Sample-36 | |

(continued)

| | | Sample and controls distribution scheme: | | | | | | | | | |
|---|---|---|---|---|---|---|---|---|---|---|---|
| | 1 | 2 | 3 | 4 | 5 | 6 | 7 | 8 | 9 | 10 | 11 | 12 |
| B | Sample-2 | | Sample-10 | | Sample-18 | | Sample-23 | | Sample-30 | | Sample-37 | |
| C | Sample-3 | | Sample-11 | | Sample-19 | | Sample-24 | | Sample-31 | | Sample-38 | |
| D | Sample-4 | | Sample-12 | | LC pool | | Sample-25 | | Sample-32 | | Sample-39 | |
| E | Sample-5 | | Sample-13 | | Control pool | | Sample-26 | | Sample-33 | | Sample-40 | |
| F | Sample-6 | | Sample-14 | | Sample-20 | | Sample-27 | | Sample-34 | | Sample-41 | |
| G | Sample-7 | | Sample-15 | | Sample-21 | | Sample-28 | | Sample-35 | | Sample-42 | |
| H | Sample-8 | | Sample-16 | | Tracer max (mAb+tracer) | | Tracer only (0 $\mu$g/ml mAb) | | | BSA | |

30 $\mu$L of PCP sample was be added to the two wells designated by "LC pool", and 30 $\mu$l of NCP sample to the two wells designated by "Control pool".

30 $\mu$l of 1X Tracer mix was added to wells H5-H10 (designated as "Tracer max" and "Tracer only").

30 $\mu$l of Blocking buffer must be added to wells H11-H12 (designated as "BSA").

Each plates were incubated then at 37°C for 60 minutes (covered by aluminum foil).

Then plasma solutions was discarded and the plates were washed for 3 times with 200 $\mu$l/well

PBS-TWEEN. 30 $\mu$L of 1:5000 diluted Streptavidin-HRP in Blocking buffer were pipetted to each well of the plates, covered and further incubated at 37°C for 30 minutes.

After 4 times washing cycles with 200 $\mu$l/well of PBS-TWEEN plates were tapped dry with paper towel to remove any excess liquid remnant from the wells. 30 $\mu$L of TMB substrate solution, prepared according to the manufacturer's instructions, were distributed from left to right to each well of the plate with an 8-channel pipette, and then covered with aluminum foil.

The enzyme reaction was halt after 2 minutes by pipetting 30 $\mu$L stop solution (4 N $H_2SO_4$) to each well with an 8-channel pipette in the similar order and speed as the TMB solution was added!

The absorbance was read at 450 nm in a microplate reader within 20 minutes after stopping the reaction.

**Initial data processing:**

First, QP biochip image analysis provided background corrected RLU values (Evidence Ivestigator Platform). After this, RLUmax% values for biochip spots incubated with plasma samples were calculated using the RLUmax data of tracer only biochip spots imaged at the same time within the same biochip rack.

In case of QP69 measurements first the quality of RLUmax measurement's data were determined. The mean, SD and CV% of the RLUmax values were calculated for each mAb. Outlier data points, which resulted CV% > 20% were excluded. The remaining data points were used to determine the average RLUmax values from the paralel measurements for each kit. In case both paralel RLUmax value were omitted, the corresponding sample RLU/RLUmax % data were omitted as well. Similar correction steps were followed in case QP300.

For QPLC21 biochip data, median, mean and variation coefficient (CV) of RLUmax% were calculated for each sample. In case of samples where the CV was above 0.25, outlier measurement's data was removed. Also, modification was done regarding of BSI variable set. From both of the QM504 and QP507 tracer datasets, Bsi0585 data was removed since during the preliminary developmental step it turned out that this mAb batch is not recognizing its original antigen. From QP507 dataset Bsi0097 and Bsi0190 variable data were also removed, since the antigens of these mAbs had been removed from the plasma protein mix during the depletion step of this tracer production. Recalculated mean of the modified RLUmax% dataset was concatenated with sample and 7 TM plus Alb. data and used for further statistical analyses.

For ELISA reading data the corresponding ODmax% values were calculated from the background corrected data. Then the mean of sample ODmax% data were used in further statistical evaluations alone or in combination with other conventional lung cancer biomarkers.

**Statistical methods:**

Corrected datasets, derived from QP69, QP300 and QPLC21 biochip or ELISA measurements, were used for further significance testing and descriptive statistical analyses utilizing different R software packages (plyr, reshape, ggplot2).

In order to build logistic regression models input variables pre-sorted by either statistical significance tests, Random Forest analysis, or Support Vector Machine with linear kernel function. Then to predict cancer, a set of the best variables were used in the binary logistic regression models with forward or sometimes with enter method. ROC analyses were also made for the predictions to detect the utility of them. During model building the tested sample cohort was divided into subsets based on histology (all and NSCLC) and tumour stage (control, early and late LC). Each models, built on a given dataset, were evaluated on all or selected possible other subsets (e.g.: All dataset, NSCLC subgroup, Control and Early LC sample pairs, Control and Late LC sample pairs, Control and Early LC sample pairs *within the* NSCLC subgroup, Control and Late LC sample pairs *within the* NSCLC subgroup etc.).

REFERENCES

1. Karu, A. E., Bell, C. W. & Chin, T. E. Recombinant Antibody Technology. ILAR J. 37, 132-141 (1995).
2. Candia, J. et al. Assessment of Variability in the SOMAscan Assay. Sci. Rep. 7, 14248 (2017).
3. Uhlén, M. et al. A Human Protein Atlas for Normal and Cancer Tissues Based on Antibody Proteomics. Mol. Cell. Proteomics 4, 1920-1932 (2005).
4. Wang, D. et al. Antigen identification and characterization of lung cancer specific monoclonal antibodies produced by mAb proteomics. J. Proteome Res. 9, 1834-1842 (2010).
5. Takacs, L. et al. Monoclonal antibody based biomarker discovery and development platform. US Patent, US 8,512,959 B2 (2005).
6. Guttman, A. & Takacs, L. Expression profiling platform technology. EU patent, EP1 802 981 B1 (2007).
7. Studer, R. A., Dessailly, B. H. & Orengo, C. A. Residue mutations and their impact on protein structure and function: detecting beneficial and pathogenic changes. Biochem. J. 449, 581-594 (2013).
8. Guergova-Kuras, M. et al. Discovery of lung cancer biomarkers by profiling the plasma proteome with monoclonal antibody libraries. Mol. {&} Cell. proteomics MCP 10, M111.010298 (2011).
9. Hajdú, I. et al. Monoclonal antibody proteomics: Use of antibody mimotope displaying phages and the relevant synthetic peptides for mAb scouting. Immunol. Lett. 160, 172-177 (2014).
10. van Klaveren, R. J. et al. Management of Lung Nodules Detected by Volume CT Scanning. N. Engl. J. Med. 361, 2221-2229 (2009).
11. Pedersen, J. H. et al. The Danish Randomized Lung Cancer CT Screening Trial-Overall Design and Results of the Prevalence Round. J. Thorac. Oncol. 4, 608-614 (2009).
12. Horeweg, N., Nackaerts, K., Oudkerk, M. & de Koning, H. J. Low-dose computed tomography screening for lung cancer: results of the first screening round. J. Comp. Eff. Res. 2, 433-436 (2013).
13. Fitzgerald, S. P., Lamont, J. V, McConnell, R. I. & Benchikh, E. O. Development of a high-throughput automated analyzer using biochip array technology. Clin. Chem. 51, 1165-76 (2005).

**Claims**

1. An *in vitro* or *ex vivo* method for detection or prognosis of lung cancer (LC) in a subject, by determination of the qualitative and quantitative variation of the antigenic epitome associated with LC, the method comprising:

   a) contacting a sample from said subject, preferably a blood plasma sample, with at least five (5) antibodies wherein

      - the first antibody specifically binds at least one epitope-antigen comprising or consisting of the sequences selected from the group consisting of SEQ ID NO: 14 to 16,
      - the second antibody specifically binds at least one epitope-antigen comprising or consisting of the sequences selected from the group consisting of SEQ ID NO: 34 to 48,
      - the third antibody specifically binds at least one epitope-antigen comprising or consisting of the sequences selected from the group consisting of SEQ ID NO: 72 to 77,
      - the fourth antibody specifically binds at least one epitope-antigen comprising or consisting of the sequences selected from the group consisting of SEQ ID NO: 109 to 114, and
      - the fifth antibody specifically binds at least one epitope-antigen comprising or consisting of the sequences selected from the group consisting of SEQ ID NO: 142 to 147,

or one fragment or derivative of such antibodies having the same antigen specificity,

b) determining the presence or the absence and the level of binding of at least the epitope-antigens of each group of sequences with said at least five antibodies, forming antibody/epitope-antigen complexes,

c) comparing the test results of the determination in step (b) to a control, whereby LC is to be detected / diagnosed / predicted, said presence or absence of the epitope-antigen complexes with said at least five antibodies being indicative of LC.

2. The method according to claim 1, wherein the step a) of the method further comprises contacting a sample from said subject, preferably a blood plasma sample, with at least one (1) antibody which specifically binds at least one epitope-antigen comprising or consisting of the sequences selected from the group consisting of SEQ ID NO: 27 to 33, or one fragment or derivative of such an antibody having the same antigen specificity.

3. The method according to claim 1, wherein the step a) of the method further comprises contacting a sample from said subject, preferably a blood plasma sample, with 15 other antibodies wherein each antibody specifically binds respectively at least one epitope-antigen of a single group comprising or consisting of the sequences selected from the groups consisting of SEQ ID NO: 1 to 5, SEQ ID NO: 6 to 13, SEQ ID NO: 17 to 19, SEQ ID NO: 20 to 26, SEQ ID NO: 49 to 59, SEQ ID NO: 60 to 71, SEQ ID NO: 78 to 82, SEQ ID NO: 83 to 95, SEQ ID NO: 96 to 108, SEQ ID NO: 115 to 126, SEQ ID NO: 127 to 135, SEQ ID NO: 136 to 141, SEQ ID NO: 148 to 154, SEQ ID NO: 155 to 172, and SEQ ID NO: 173 to 180, or one fragment or derivative of such an antibody having the same antigen specificity.

4. The method according to claim 1 or 2, wherein the step a) of the method comprises contacting a sample from said subject, preferably a blood plasma sample, with six (6) antibodies wherein

- the first antibody specifically binds at least one epitope-antigen comprising or consisting of the sequences selected from the group consisting of SEQ ID NO: 14 to 16,
- the second antibody specifically binds at least one epitope-antigen comprising or consisting of the sequences selected from the group consisting of SEQ ID NO: 34 to 48,
- the third antibody specifically binds at least one epitope-antigen comprising or consisting of the sequences selected from the group consisting of SEQ ID NO: 72 to 77,
- the fourth antibody specifically binds at least one epitope-antigen comprising or consisting of the sequences selected from the group consisting of SEQ ID NO: 109 to 114,
- the fifth antibody specifically binds at least one epitope-antigen comprising or consisting of the sequences selected from the group consisting of SEQ ID NO: 142 to 147, and
- the sixth antibody specifically binds at least one epitope-antigen comprising or consisting of the sequences selected from the group consisting of SEQ ID NO: 27 to 33, or one fragment or derivative of such antibodies having the same antigen specificity.

5. The method according to claim 1 or 3, wherein the step a) of the method comprises contacting a sample from said subject, preferably a blood plasma sample, with twenty (20) antibodies wherein each antibody specifically binds respectively at least one epitope-antigen of a single group comprising or consisting of the sequences selected from the groups consisting of SEQ ID NO: 1 to 5, SEQ ID NO: 6 to 13, SEQ ID NO: 14 to 16, SEQ ID NO: 17 to 19, SEQ ID NO: 20 to 26, SEQ ID NO: 34 to 48, SEQ ID NO: 49 to 59, SEQ ID NO: 60 to 71, SEQ ID NO: 72 to 77, SEQ ID NO: 78 to 82, SEQ ID NO: 83 to 95, SEQ ID NO: 96 to 108, SEQ ID NO: 109 to 114, SEQ ID NO: 115 to 126, SEQ ID NO: 127 to 135, SEQ ID NO: 136 to 141, SEQ ID NO: 142 to 147, SEQ ID NO: 148 to 154, SEQ ID NO: 155 to 172, and SEQ ID NO: 173 to 180, or one fragment or derivative of such an antibody having the same antigen specificity.

6. The method according to any one of claims 1 to 5, wherein in step a) of the method

each antibody is immobilized directly to a support or captured by an affinity reagent such as an anti-mouse IgG antibody coated onto a support,
each immobilized antibody is then incubated with the sample from said subject.

7. The method according to any one of claims 1 to 6, wherein in step b) each immobilized antibody/epitope-antigen complex is then incubated with a labeled tracer consisting of either (i) labeled proteins which contain the epitope-antigens with a detection molecule such as biotin, or (ii) a purified or recombinant protein recognized by the monoclonal antibody, or (iii) an epitope-antigen which is recognized by the monoclonal antibody, and is detected by the addition of a reagent which binds to the label such as avidin or streptavidin.

8. The method according to any one of claims 1 to 7, wherein the method further comprises

   d) calculating a probability index based on logistic regression models with forward or enter method; and
   e) determining the probability of the presence or absence of LC in the subject, based on the probability index.

9. The method according to any one of claims 1 to 8, wherein the method further comprises determining the level of biomarkers chosen among the group consisting of albumin, CA125, CEA, CYFRA, TPAM, CRP, HE4 and CRP, in particular CYFRA.

10. The method according to any one of claims 1 to 9, to profile samples from subjects chosen among healthy subjects, stage I LC, stage II LC, stage III LC, stage IV LC, relapsing LC.

11. The method according to any one of claims 1 to 10, wherein the method is able to discern subjects that do not have LC but have one or more symptoms associated with LC, from subjects having LC, with a receiver operator characteristic (ROC) AUC value of at least 0.75 more preferably 0.80, 0.85 or 0.90.

12. The method according to any one of claims 1 to 11, wherein the method is able to discern subjects not having LC but having one or more symptoms associated with LC, from subjects having LC, with specificity of at least 80% more preferably 85%, 90% or 95%.

13. A device for diagnosing, providing prognosis, monitoring, characterizing, determining a severity of, confirming a presence of, or confirming an absence of LC in a subject, the device comprising:

   a) an input module for receiving as an input levels of one or more epitope-antigen;
   (b) a processor configured to: perform an algorithm, based on logistic regression models with forward or enter method, adjusting the levels of each of the one or more epitope-antigen to a corresponding control value, thereby providing one or more epitope-antigen levels; perform a real function algorithm manipulating the one or more epitope-antigen levels by multiplying one or more variables by one or more corresponding weight factors, wherein a level of each of the one or more adjusted epitope-antigen levels is input into a specific variable of the one or more variables, wherein the corresponding weight factor is unique for each specific variable, wherein at least one of the corresponding weight factors is different from one; and
   (c) an output module for outputting an index value, wherein the index value indicates diagnosis, prognosis, characterization, a monitored aspect, determination of the severity, confirmation of the presence, or confirmation of the absence, of LC in the subject, wherein the device comprises at least five (5) antibodies wherein

   - the first antibody specifically binds at least one epitope-antigen comprising or consisting of the sequences selected from the group consisting of SEQ ID NO: 14 to 16,
   - the second antibody specifically binds at least one epitope-antigen comprising or consisting of the sequences selected from the group consisting of SEQ ID NO: 34 to 48,
   - the third antibody specifically binds at least one epitope-antigen comprising or consisting of the sequences selected from the group consisting of SEQ ID NO: 72 to 77,
   - the fourth antibody specifically binds at least one epitope-antigen comprising or consisting of the sequences selected from the group consisting of SEQ ID NO: 109 to 114, and
   - the fifth antibody specifically binds at least one epitope-antigen comprising or consisting of the sequences selected from the group consisting of SEQ ID NO: 142 to 147,

   or one fragment or derivative of such antibodies having the same antigen specificity.

**Patentansprüche**

1. *In-vitro-* oder *ex-vivo*-Verfahren zum Nachweis oder zur Prognose von Lungenkrebs (LC) in einem Individuum durch Bestimmung der qualitativen und quantitativen Variation des mit LC assoziierten antigenen Epitoms, wobei das Verfahren umfasst:

   a) Inkontaktbringen einer Probe von dem Individuum, vorzugsweise einer Blutplasmaprobe, mit wenigstens fünf (5) Antikörpern, wobei

- der erste Antikörper spezifisch wenigstens ein Epitop-Antigen bindet, umfassend oder bestehend aus den Sequenzen, die aus der Gruppe ausgewählt sind, die aus SEQ ID NO: 14 bis 16 besteht,
- der zweite Antikörper spezifisch wenigstens ein Epitop-Antigen bindet, umfassend oder bestehend aus den Sequenzen, die aus der Gruppe ausgewählt sind, die aus SEQ ID NO: 34 bis 48 besteht,
- der dritte Antikörper spezifisch wenigstens ein Epitop-Antigen bindet, umfassend oder bestehend aus den Sequenzen, die aus der Gruppe ausgewählt sind, die aus SEQ ID NO: 72 bis 77 besteht,
- der vierte Antikörper spezifisch wenigstens ein Epitop-Antigen bindet, umfassend oder bestehend aus den Sequenzen, die aus der Gruppe ausgewählt sind, die aus SEQ ID NO: 109 bis 114 besteht, und
- der fünfte Antikörper spezifisch wenigstens ein Epitop-Antigen bindet, umfassend oder bestehend aus den Sequenzen, die aus der Gruppe ausgewählt sind, die aus SEQ ID NO: 142 bis 147 besteht, oder einem Fragment oder Derivat solcher Antikörper mit der gleichen Antigen-Spezifizität,

b) Bestimmen der Gegenwart oder Abwesenheit und des Bindungsniveaus wenigstens der Epitop-Antigene jeder Gruppe von Sequenzen mit den wenigstens fünf Antikörpern, die Antikörper/Epitop-Antigen-Komplexe bilden,
c) Vergleichen der Testergebnisse der Bestimmung in Schritt (b) mit einer Kontrolle, wodurch LC nachgewiesen / diagnostiziert / vorhergesagt werden kann, wobei die Gegenwart oder Abwesenheit der Epitop-Antigen-Komplexe mit den wenigstens fünf Antikörpern ein Hinweis auf LC ist.

2.  Verfahren nach Anspruch 1, wobei der Schritt a) des Verfahrens ferner umfasst: das Inkontaktbringen einer Probe von dem Individuum, vorzugsweise einer Blutplasmaprobe, mit wenigstens einem (1) Antikörper, der spezifisch wenigstens ein Epitop-Antigen bindet, umfassend oder bestehend aus den Sequenzen, die aus der Gruppe ausgewählt sind, die aus SEQ ID NO: 27 bis 33 besteht, oder einem Fragment oder Derivat eines solchen Antikörpers mit der gleichen Antigenspezifizität.

3.  Verfahren nach Anspruch 1, wobei der Schritt a) des Verfahrens ferner umfasst: das Inkontaktbringen einer Probe von dem Individuum, vorzugsweise einer Blutplasmaprobe, mit 15 anderen Antikörpern, wobei jeder Antikörper spezifisch jeweils wenigstens ein Epitop-Antigen einer einzelnen Gruppe bindet, umfassend oder bestehend aus den Sequenzen, die aus den Gruppen ausgewählt sind, die aus SEQ ID NO: 1 bis 5, SEQ ID NO: 6 bis 13, SEQ ID NO: 17 bis 19, SEQ ID NO: 20 bis 26, SEQ ID NO: 49 bis 59, SEQ ID NO: 60 bis 71, SEQ ID NO: 78 bis 82, SEQ ID NO: 83 bis 95, SEQ ID NO: 96 bis 108, SEQ ID NO: 115 bis 126, SEQ ID NO: 127 bis 135, SEQ ID NO: 136 bis 141, SEQ ID NO: 148 bis 154, SEQ ID NO: 155 bis 172 und SEQ ID NO: 173 bis 180 bestehen, oder einem Fragment oder Derivat eines solchen Antikörpers mit der gleichen Antigenspezifizität.

4.  Verfahren nach Anspruch 1 oder 2, wobei der Schritt a) des Verfahrens umfasst: das Inkontaktbringen einer Probe von dem Individuum, vorzugsweise einer Blutplasmaprobe, mit sechs (6) Antikörpern, wobei

- der erste Antikörper spezifisch wenigstens ein Epitop-Antigen bindet, umfassend oder bestehend aus den Sequenzen, die aus der Gruppe ausgewählt sind, die aus SEQ ID NO: 14 bis 16 besteht,
- der zweite Antikörper spezifisch wenigstens ein Epitop-Antigen bindet, umfassend oder bestehend aus den Sequenzen, die aus der Gruppe ausgewählt sind, die aus SEQ ID NO: 34 bis 48 besteht,
- der dritte Antikörper spezifisch wenigstens ein Epitop-Antigen bindet, umfassend oder bestehend aus den Sequenzen, die aus der Gruppe ausgewählt sind, die aus SEQ ID NO: 72 bis 77 besteht,
- der vierte Antikörper spezifisch wenigstens ein Epitop-Antigen bindet, umfassend oder bestehend aus den Sequenzen, die aus der Gruppe ausgewählt sind, die aus SEQ ID NO: 109 bis 114 besteht,
- der fünfte Antikörper spezifisch wenigstens ein Epitop-Antigen bindet, umfassend oder bestehend aus den Sequenzen, die aus der Gruppe ausgewählt sind, die aus SEQ ID NO: 142 bis 147 besteht, und
- der sechste Antikörper spezifisch wenigstens ein Epitop-Antigen bindet, umfassend oder bestehend aus den Sequenzen, die aus der Gruppe ausgewählt sind, die aus SEQ ID NO: 27 bis 33 besteht, oder einem Fragment oder Derivat solcher Antikörper mit der gleichen Antigen-Spezifizität.

5.  Verfahren nach Anspruch 1 oder 3, wobei der Schritt a) des Verfahrens umfasst: das Inkontaktbringen einer Probe von dem Individuum, vorzugsweise einer Blutplasmaprobe, mit zwanzig (20) Antikörpern, wobei jeder Antikörper spezifisch jeweils wenigstens ein Epitop-Antigen einer einzigen Gruppe bindet, umfassend oder bestehend aus den Sequenzen, die aus den Gruppen ausgewählt sind, die aus SEQ ID NO: 1 bis 5, SEQ ID NO: 6 bis 13, SEQ ID NO: 14 bis 16, SEQ ID NO: 17 bis 19, SEQ ID NO: 20 bis 26, SEQ ID NO: 34 bis 48, SEQ ID NO: 49 bis 59, SEQ ID NO: 60 bis 71, SEQ ID NO: 72 bis 77, SEQ ID NO: 78 bis 82, SEQ ID NO: 83 bis 95, SEQ ID NO: 96 bis 108, SEQ ID NO: 109 bis 114, SEQ ID NO: 115 bis 126, SEQ ID NO: 127 bis 135, SEQ ID NO: 136 bis 141, SEQ ID NO: 142 bis 147, SEQ ID NO: 148 bis 154, SEQ ID NO: 155 bis 172 und SEQ ID NO: 173 bis 180 bestehen, oder einem Fragment oder Derivat eines solchen Antikörpers mit der gleichen Antigenspezifizität.

6. Verfahren nach einem der Ansprüche 1 bis 5, wobei in Schritt a) des Verfahrens jeder Antikörper direkt auf einem Träger immobilisiert wird oder durch ein Affinitätsreagens wie z. B. einen Anti-Maus-IgG-Antikörper, der auf einen Träger aufgebracht ist, gefangen wird, wobei jeder immobilisierte Antikörper anschließend mit der Probe von dem Individuum inkubiert wird.

7. Verfahren nach einem der Ansprüche 1 bis 6, wobei in Schritt b) jeder immobilisierte Antikörper/Epitop-Antigen-Komplex anschließend mit einem markierten Tracer inkubiert wird, der aus Folgendem besteht: entweder (i) aus markierten Proteinen, die die Epitop-Antigene mit einem Nachweismolekül, wie z. B. Biotin, enthalten, oder (ii) aus einem gereinigten oder rekombinanten Protein, das durch den monoklonalen Antikörper erkannt wird, oder (iii) aus einem Epitop-Antigen, das durch den monoklonalen Antikörper erkannt wird, und durch die Zugabe eines Reagens nachgewiesen wird, das an die Markierung bindet, wie z. B. Avidin oder Streptavidin.

8. Verfahren nach einem der Ansprüche 1 bis 7, wobei das Verfahren ferner umfasst:

   d) Berechnen eines Wahrscheinlichkeitsindex auf der Grundlage logistischer Regressionsmodelle mit Vorwärts- oder Eingabeverfahren; und
   e) Bestimmen der Wahrscheinlichkeit der Gegenwart oder Abwesenheit von LC in dem Individuum auf der Grundlage des Wahrscheinlichkeitsindex.

9. Verfahren nach einem der Ansprüche 1 bis 8, wobei das Verfahren ferner das Bestimmen des Spiegels von Biomarkern umfasst, die aus der Gruppe ausgewählt sind, die aus Albumin, CA125, CEA, CYFRA, TPAM, CRP, HE4 und CRP besteht, insbesondere CYFRA.

10. Verfahren nach einem der Ansprüche 1 bis 9 zur Profilierung von Proben von Individuen, die ausgewählt sind aus gesunden Individuen, solchen mit LC im Stadium I, LC im Stadium II, LC im Stadium III, LC im Stadium IV, rezidivierendem LC.

11. Verfahren nach einem der Ansprüche 1 bis 10, wobei das Verfahren imstande ist, Individuen, die keinen LC haben, aber ein oder mehrere Symptome aufweisen, die mit LC assoziiert sind, mit einem AUC-Wert der Operationscharakteristik eines Beobachters (ROC) von wenigstens 0,75, stärker bevorzugt 0,80, 0,85 oder 0,90, von Individuen zu unterscheiden, die LC haben.

12. Verfahren nach einem der Ansprüche 1 bis 11, wobei das Verfahren imstande ist, Individuen, die keinen LC haben, aber ein oder mehrere Symptome aufweisen, die mit LC assoziiert sind, mit einer Spezifizität von wenigstens 80 %, stärker bevorzugt 85 %, 90 % oder 95 %, von Individuen zu unterscheiden, die LC haben.

13. Vorrichtung zum Diagnostizieren, Bereitstellen einer Diagnose, Überwachen, Charakterisieren, Bestimmen eines Schweregrads, Bestätigen einer Gegenwart oder Bestätigen einer Abwesenheit von LC in einem Individuum, wobei die Vorrichtung umfasst:

   a) ein Eingabemodul zum Empfangen der Spiegel von oder einem oder mehreren Epitop-Antigenen als Eingang;
   b) einen Prozessor, der für Folgendes ausgelegt ist: Ausführen eines Algorithmus auf der Grundlage logistischer Regressionsmodelle mit Vorwärts-oder Eingabeverfahren, Einstellen der Spiegel von jedem des einen oder der mehreren Epitop-Antigene auf einen entsprechenden Kontrollwert, wodurch ein oder mehrere Epitop-Antigen-Spiegel bereitgestellt werden; Durchführen eines realen Funktionsalgorithmus, der den einen oder die mehreren Epitop-Antigen-Spiegel manipuliert, indem ein oder mehrere Variablen mit einem oder mehreren entsprechenden Gewichtsfaktoren multipliziert werden, wobei ein Spiegel von jedem des einen oder der mehreren eingestellten Epitop-Antigen-Spiegel in eine spezifische Variable der einen oder mehreren Variablen eingegeben wird, wobei der entsprechende Gewichtsfaktor für jede spezifische Variable einzigartig ist, wobei wenigstens einer der entsprechenden Gewichtsfaktoren ein anderer als eins ist; und
   c) ein Ausgabemodul zur Ausgabe eines Indexwerts, wobei der Indexwert die Diagnose, die Prognose, die Charakterisierung, einen überwachten Aspekt, die Bestimmung des Schweregrads, die Bestätigung der Gegenwart oder die Bestätigung der Abwesenheit von LC in dem Individuum angibt, wobei die Vorrichtung umfasst: wenigstens fünf (5) Antikörper, wobei

- der erste Antikörper spezifisch wenigstens ein Epitop-Antigen bindet, umfassend oder bestehend aus den Sequenzen, die aus der Gruppe ausgewählt sind, die aus SEQ ID NO: 14 bis 16 besteht,
- der zweite Antikörper spezifisch wenigstens ein Epitop-Antigen bindet, umfassend oder bestehend aus den Sequenzen, die aus der Gruppe ausgewählt sind, die aus SEQ ID NO: 34 bis 48 besteht,
- der dritte Antikörper spezifisch wenigstens ein Epitop-Antigen bindet, umfassend oder bestehend aus den Sequenzen, die aus der Gruppe ausgewählt sind, die aus SEQ ID NO: 72 bis 77 besteht,
- der vierte Antikörper spezifisch wenigstens ein Epitop-Antigen bindet, umfassend oder bestehend aus den Sequenzen, die aus der Gruppe ausgewählt sind, die aus SEQ ID NO: 109 bis 114 besteht, und
- der fünfte Antikörper spezifisch wenigstens ein Epitop-Antigen bindet, umfassend oder bestehend aus den Sequenzen, die aus der Gruppe ausgewählt sind, die aus SEQ ID NO: 142 bis 147 besteht,

oder ein Fragment oder Derivat solcher Antikörper mit der gleichen Antigen-Spezifizität.

**Revendications**

1. Méthode *in vitro* ou *ex vivo* de détection ou de pronostic d'un cancer du poumon (LC) chez un sujet, par détermination de la variation qualitative et quantitative de l'épitome antigénique associé au LC, la méthode comprenant :

   a) la mise en contact d'un échantillon dudit sujet, de préférence un échantillon de plasma sanguin, avec au moins cinq (5) anticorps dans lesquels

   - le premier anticorps se lie spécifiquement à au moins un épitope-antigène comprenant ou constitué par les séquences sélectionnées dans le groupe constitué par SEQ ID NO : 14 à 16,
   - le second anticorps se lie spécifiquement à au moins un épitope-antigène comprenant ou constitué des séquences sélectionnées dans le groupe constitué par SEQ ID NO : 34 à 48,
   - le troisième anticorps se lie spécifiquement à au moins un épitope-antigène comprenant ou constitué par les séquences sélectionnées dans le groupe constitué par SEQ ID NO : 72 à 77,
   - le quatrième anticorps se lie spécifiquement à au moins un épitope-antigène comprenant ou constitué des séquences sélectionnées dans le groupe constitué par SEQ ID NO : 109 à 114, et
   - le cinquième anticorps se lie spécifiquement à au moins un épitope-antigène comprenant ou constitué des séquences sélectionnées dans le groupe constitué par SEQ ID NO : 142 à 147,

   ou un fragment ou un dérivé de ces anticorps ayant la même spécificité antigénique,
   b) la détermination de la présence ou de l'absence et le niveau de liaison d'au moins les épitopes-antigènes de chaque groupe de séquences avec lesdits au moins cinq anticorps, formant des complexes anticorps/épitopes-antigènes,
   c) la comparaison des résultats des tests de la détermination de l'étape (b) à un témoin, par lequel le LC doit être détecté / diagnostiqué / prédit, ladite présence ou absence des complexes épitopes-antigènes avec lesdits au moins cinq anticorps indiquant le LC.

2. Méthode selon la revendication 1, dans laquelle l'étape a) de la méthode consiste en outre en une mise en contact d'un échantillon dudit sujet, de préférence un échantillon de plasma sanguin, avec au moins un (1) anticorps qui se lie spécifiquement à au moins un épitope-antigène comprenant ou consistant en les séquences sélectionnées dans le groupe constitué par SEQ ID NO : 27 à 33, ou un fragment ou un dérivé d'un tel anticorps ayant la même spécificité antigénique.

3. Méthode selon la revendication 1, dans laquelle l'étape a) de la méthode consiste en outre en une mise en contact d'un échantillon dudit sujet, de préférence un échantillon de plasma sanguin, avec 15 autres anticorps, chaque anticorps se liant spécifiquement à au moins un épitope-antigène d'un seul groupe comprenant ou constitué des séquences sélectionnées parmi les groupes constitués de SEQ ID NO : 1 à 5, SEQ ID NO : 6 à 13, SEQ ID NO : 17 à 19, SEQ ID NO : 20 à 26, SEQ ID NO : 49 à 59, SEQ ID NO : 60 à 71, SEQ ID NO : 78 à 82, SEQ ID NO : 83 à 95, SEQ ID NO : 96 à 108, SEQ ID NO : 115 à 126, SEQ ID NO : 127 à 135, SEQ ID NO : 136 à 141, SEQ ID NO : 148 à 154, SEQ ID NO : 155 à 172 et SEQ ID NO : 173 à 180, ou un fragment ou un dérivé d'un tel anticorps ayant la même spécificité antigénique.

4. Méthode selon la revendication 1 ou 2, dans laquelle l'étape a) de la méthode consiste en la mise en contact d'un échantillon dudit sujet, de préférence un échantillon de plasma sanguin, avec six (6) anticorps dans lesquels

- le premier anticorps se lie spécifiquement à au moins un épitope-antigène comprenant ou constitué par les séquences sélectionnées dans le groupe constitué par SEQ ID NO : 14 à 16,

- le second anticorps se lie spécifiquement à au moins un épitope-antigène comprenant ou constitué des séquences sélectionnées dans le groupe constitué par SEQ ID NO : 34 à 48,

- le troisième anticorps se lie spécifiquement à au moins un épitope-antigène comprenant ou constitué par les séquences sélectionnées dans le groupe constitué par SEQ ID NO : 72 à 77,

- le quatrième anticorps se lie spécifiquement à au moins un épitope-antigène comprenant ou consistant en les séquences sélectionnées dans le groupe constitué par SEQ ID NO : 109 à 114,

- le cinquième anticorps se lie spécifiquement à au moins un épitope-antigène comprenant ou constitué des séquences sélectionnées dans le groupe constitué par SEQ ID NO : 142 à 147, et

- le sixième anticorps se lie spécifiquement à au moins un épitope-antigène comprenant ou consistant en les séquences sélectionnées dans le groupe constitué par SEQ ID NO : 27 à 33,

ou un fragment ou un dérivé de ces anticorps ayant la même spécificité antigénique.

5. Méthode selon la revendication 1 ou 3, dans laquelle l'étape a) de la méthode consiste en la mise en contact d'un échantillon dudit sujet, de préférence un échantillon de plasma sanguin, avec vingt (20) anticorps, chaque anticorps se liant spécifiquement respectivement à au moins un épitope-antigène d'un seul groupe comprenant ou constitué des séquences sélectionnées parmi les groupes constitués de SEQ ID NO : 1 à 5, SEQ ID NO : 6 à 13, SEQ ID NO : 14 à 16, SEQ ID NO : 17 à 19, SEQ ID NO : 20 à 26, SEQ ID NO : 34 à 48, SEQ ID NO : 49 à 59, SEQ ID NO : 60 à 71, SEQ ID NO : 72 à 77, SEQ ID NO : 78 à 82, SEQ ID NO : 83 à 95, SEQ ID NO : 96 à 108, SEQ ID NO : 109 à 114, SEQ ID NO : 115 à 126, SEQ ID NO : 127 à 135, SEQ ID NO : 136 à 141, SEQ ID NO : 142 à 147, SEQ ID NO : 148 à 154, SEQ ID NO : 155 à 172 et SEQ ID NO : 173 à 180, ou un fragment ou un dérivé d'un tel anticorps ayant la même spécificité antigénique.

6. Méthode selon l'une quelconque des revendications 1 à 5, dans laquelle à l'étape a) de la méthode chaque anticorps est immobilisé directement sur un support ou capturé par un réactif d'affinité tel qu'un anticorps IgG anti-souris appliqué sur un support, chaque anticorps immobilisé est ensuite incubé avec l'échantillon dudit sujet.

7. Méthode selon l'une quelconque des revendications 1 à 6, dans laquelle à l'étape b) chaque complexe anticorps/épitope-antigène immobilisé est ensuite incubé avec un traceur marqué composé soit (i) de protéines marquées qui contiennent les épitopes-antigènes avec une molécule de détection telle que la biotine, soit (ii) d'une protéine purifiée ou recombinante reconnue par l'anticorps monoclonal, soit (iii) d'un épitope-antigène reconnu par l'anticorps monoclonal, et détecté par l'ajout d'un réactif qui se lie au marqueur tel que l'avidine ou la streptavidine.

8. Méthode selon l'une quelconque des revendications 1 à 7, dans laquelle la méthode comprend en outre

d) le calcul d'un indice de probabilité basé sur des modèles de régression logistique avec la méthode forward ou enter ; et

e) la détermination de la probabilité de la présence ou de l'absence de LC chez le sujet, sur la base de l'indice de probabilité.

9. Méthode selon l'une quelconque des revendications 1 à 8, dans laquelle la méthode comprend en outre la détermination du niveau de biomarqueurs choisis parmi le groupe constitué par l'albumine, le CA125, le CEA, le CYFRA, le TPAM, le CRP, l'HE4 et le CRP, en particulier le CYFRA.

10. Méthode selon l'une quelconque des revendications 1 à 9, pour profiler des échantillons provenant de sujets choisis parmi des sujets sains, de stade I du LC, de stade II du LC, de stade III du LC, de stade IV du LC, récidivant du LC.

11. Méthode selon l'une quelconque des revendications 1 à 10, dans laquelle la méthode est capable de discerner des sujets qui n'ont pas de LC mais qui présentent un ou plusieurs symptômes associés au LC, des sujets ayant un LC, avec une valeur ASC de la fonction d'efficacité du récepteur (ROC) d'au moins 0,75 de préférence 0,80, 0,85 ou 0,90.

12. Méthode selon l'une quelconque des revendications 1 à 11, dans laquelle la méthode est capable de discerner des sujets n'ayant pas de LC mais présentant un ou plusieurs symptômes associés au LC, des sujets ayant un LC, avec une spécificité d'au moins 80 % plus de préférence 85 %, 90 % ou 95 %.

**13.** Dispositif de diagnostic, de pronostic, de surveillance, de caractérisation, de détermination de la gravité, de confirmation de la présence ou de confirmation d'une absence de LC chez un sujet, le dispositif comprenant :

a) un module d'entrée pour recevoir en entrée des niveaux d'un ou de plusieurs épitopes-antigènes;
b) un processeur configuré pour : exécuter un algorithme, basé sur des modèles de régression logistique avec la méthode forward ou input, ajustant les niveaux de chacun des un ou plusieurs épitopes-antigènes à une valeur de contrôle correspondante, fournissant ainsi un ou plusieurs niveaux d'épitopes-antigènes ; exécuter un algorithme de fonction réelle manipulant les niveaux d'un ou de plusieurs épitopes-antigènes en multipliant une ou plusieurs variables par un ou plusieurs facteurs de pondération correspondants, dans lequel un niveau de chacun des niveaux d'épitopes-antigènes ajustés est entré dans une variable spécifique d'une ou plusieurs variables, dans laquelle le facteur de pondération correspondant est unique pour chaque variable spécifique, dans laquelle au moins un des facteurs de pondération correspondants est différent de un ; et
c) un module de sortie pour la production d'une valeur d'indice, dans lequel la valeur d'indice indique le diagnostic, le pronostic, la caractérisation, un aspect surveillé, la détermination de la gravité, la confirmation de la présence ou la confirmation de l'absence de LC chez le sujet, dans lequel le dispositif comprend au moins cinq (5) anticorps dans lesquels

- le premier anticorps se lie spécifiquement à au moins un épitope-antigène comprenant ou constitué par les séquences sélectionnées dans le groupe constitué par SEQ ID NO : 14 à 16,
- le second anticorps se lie spécifiquement à au moins un épitope-antigène comprenant ou constitué des séquences sélectionnées dans le groupe constitué par SEQ ID NO : 34 à 48,
- le troisième anticorps se lie spécifiquement à au moins un épitope-antigène comprenant ou constitué par les séquences sélectionnées dans le groupe constitué par SEQ ID NO : 72 à 77,
- le quatrième anticorps se lie spécifiquement à au moins un épitope-antigène comprenant ou constitué des séquences sélectionnées dans le groupe constitué par SEQ ID NO : 109 à 114, et
- le cinquième anticorps se lie spécifiquement à au moins un épitope-antigène comprenant ou constitué des séquences sélectionnées dans le groupe constitué par SEQ ID NO : 142 à 147,

ou un fragment ou un dérivé de ces anticorps ayant la même spécificité antigénique.

A

**RLUmax**

**RLU/RLUmax%**

**RLU/RLUmax%**

• 25 experimental days – tracer only sample
• Measured twice/day – daily mean RLUmax
• Mean and SD of the 25 daily mean RLUmax values are calculated
• **CV% = Mean / SD * 100 (%)**
• 69 mAbs – 69 CV% values

• 10 experimental days – 1 pooled plasma – 2 dilutions (100x, 1000x)
• Measured once/day – RLU/RLUmax% value is calculated
• Mean and SD of the 10 RLU/RLUmax% values are calculated
• **CV% = Mean / SD * 100 (%)**
• 69 mAbs – 69 CV% values

$y = 1030x + 27,7$
$R = 0,998$

LOT 4381
• 14 experimental days – tracer only sample
• Measured twice/day – daily mean RLUmax
• Mean of the 14 daily mean RLUmax values are calculated
• 1 square = 1 mAb

LOT 4383
• 25 experimental days – tracer only sample
• Measured twice/day – daily mean RLUmax
• Mean of the 25 daily mean RLUmax values are calculated
• 1 square = 1 mAb

B

<u>Figure 1</u>

**Figure 2**

A

ROC Curve

D

**Area Under the Curve**

| Area | Std. Error[a] | Asymptotic Sig.[b] | Asymptotic 95% Confidence Interval | |
|---|---|---|---|---|
| | | | Lower Bound | Upper Bound |
| 0,842 | 0,011 | 0,000 | 0,820 | 0,865 |

a. Under the nonparametric assumption

b. Null hypothesis: true area = 0.5

| Sensitivity | Specificity |
|---|---|
| 100,0 | 5,7 |
| 95,0 | 35,6 |
| 90,0 | 51,2 |
| 80,0 | 71,1 |
| 73,1 | 80,0 |
| 58,4 | 90,0 |
| 43,9 | 95,0 |
| 1,3 | 100,0 |

B Bsi0097, Bsi0116, Bsi0142, Bsi0144, Bsi0186, Bsi0190, Bsi0221, Bsi0439, Bsi0581, Bsi0585, Bsi0639, Bsi0686, Bsi0759, Bsi0789, Bsi2487, Bsi1154, Bsi1186, Bsi1517, Bsi1328, Bsi0300, Bsi0782

E

| | N | % |
|---|---|---|
| Control | 598 | 51,7 |
| Tumor | 558 | 48,3 |
| Total | 1156 | 100,0 |

C $\hat{P}$=(1/(1+e^-(0.754+0,069*Bsi0097_800x-0.007*Bsi0116_800x
-0.002*Bsi0142_800x+0.02*Bsi0144_800x-0.07*Bsi0186_800x-0.002*Bsi0190_800x
+0.048*Bsi0221_800x+0.043*Bsi0439_800x-0.085*Bsi0581_800x-0.035*Bsi0585_800x
+0.017*Bsi0639_800x+0.045*Bsi0686_800x+0.042*Bsi0759_800x-0.033*Bsi0789_800x
+0.053*Bsi2487_800x-0.116*Bsi1154_800x+0.04*Bsi1186_800x-0.014*Bsi1517_800x-
0.008*Bsi1328_800x+0.033*Bsi0300_800x-0.009*Bsi0782_800x)))*100

**Figure 3**

**A**

ROC Curve

Sensitivity (y-axis), 1 - Specificity (x-axis)

Extended M61 model - QM504 group

**B** Bsi0144 (ACT), Bsi0581 (C9), Bsi0186 (ACT), Bsi2487
(Gelsolin), Bsi0190 (C3), Bsi0782 (C5), CYFRA, CRP,
Albumin

**C** $\hat{P}$ =(1/(1+e^-(1.423+0.059*Bsi0144-0.084*Bsi0581-
0.066*Bsi0186+0.064*Bsi2487-0,04*Bsi0190
+0.085*CYFRA+0.004*CRP-0.003*Albumin)))*100

**D**

Area Under the Curve

Test Result Variable(s): M61ext

| Area | Std. Error[a] | Asymptotic Sig.[b] | Asymptotic 95% Confidence Interval | |
|---|---|---|---|---|
| | | | Lower Bound | Upper Bound |
| ,820 | ,024 | ,000 | ,772 | ,868 |

a. Under the nonparametric assumption

b. Null hypothesis: true area = 0.5

| Sensitivity | Specificity |
|---|---|
| 100,0 | 0,80 |
| 95,0 | 19,20 |
| 90,0 | 53,60 |
| 80,0 | 74,40 |
| 69,0 | 80,0 |
| 52,7 | 90,0 |
| 39,7 | 95,0 |
| 9,2 | 100,0 |

**E**

| Sample type | Count | Age average |
|---|---|---|
| Control | 125 | 62.58 |
| LC | 185 | 62.45 |
| C_COPD_BD | 99 | 62.66 |
| C_NonCOPD_BD | 26 | 62.31 |
| LC_COPD_BD | 124 | 63.01 |
| LC_NonCOPD_BD | 61 | 61.33 |

**Figure 4**

A

**ROC Curve**

Extended M63 model - QM504-QP507 group

B  $P^*=(1/(1+e^{\wedge}(5.021+0.022{*}QM\_Bsi0144-0,071{*}QP\_Bsi0581 +0.026{*}QP\_Bsi2487-0,017{*}QM\_Bsi0116+0,006{*}QP\_Bsi0782 -0.019{*}QM\_Bsi0581-0,043{*}QP\_Bsi0116-0,027{*}QM\_Bsi0186 -0.025{*}QP\_Bsi1186+0.009{*}QP\_Bsi0759+0.052{*}QP\_Bsi0221 +0.080{*}CYFRA +0.004{*} CRP+0.009{*}Albumin)))*100$

C  QM_Bsi0144, QP_Bsi0581, QP_Bsi2487, QM_Bsi0116, QP_Bsi0782, QM_Bsi0581, QP_Bsi0116, QM_Bsi0186, QP_Bsi1186, QP_Bsi0759, QP_Bsi0221, CYFRA, CRP and Albumin

D

**Area Under the Curve**

Test Result Variable(s): M63ext

| Area | Std. Error[a] | Asymptotic Sig.[b] | Asymptotic 95% Confidence Interval | |
|---|---|---|---|---|
| | | | Lower Bound | Upper Bound |
| ,794 | ,025 | ,000 | ,745 | ,844 |

a. Under the nonparametric assumption

b. Null hypothesis: true area = 0.5

| Sensitivity | Specificity |
|---|---|
| 100,0 | 1,60 |
| 95,0 | 27,20 |
| 90,0 | 42,40 |
| 80,0 | 60,80 |
| 63,8 | 80,0 |
| 52,4 | 90,0 |
| 35,7 | 95,0 |
| 15,1 | 100,0 |

E

| Sample type | Count | Age average |
|---|---|---|
| Control | 125 | 62.58 |
| LC | 185 | 62.45 |
| C_COPD_BD | 99 | 62.66 |
| C_NonCOPD_BD | 26 | 62.31 |
| LC_COPD_BD | 124 | 63.01 |
| LC_NonCOPD_BD | 61 | 61.33 |

**Figure 5**

A   Bsi0581 (C9), Bsi0782 (C5), Bsi0759 (Alpha2HS), Bsi1186 (C4BP), CYFRA

B   $$\widehat{P} = \left( \frac{1}{1 + e^{(1.746 + 0.149*CYFRA - 0.061*QP\_Bsi0581 - 0.056*QP\_Bsi1186 + 0.017*QP\_Bsi0759 + 0.045*QP\_Bsi0782)}} \right) * 100$$

C

### All

**Area Under the Curve**

Test Result Variable(s): M48

| Area | Std. Error[a] | Asymptotic Sig.[b] | Asymptotic 95% Confidence Interval | |
|---|---|---|---|---|
| | | | Lower Bound | Upper Bound |
| ,763 | ,027 | ,000 | ,710 | ,815 |

The test result variable(s): M48 has at least one tie between the positive actual state group and the negative actual state group. Statistics may be biased.

a. Under the nonparametric assumption

b. Null hypothesis: true area = 0.5

| Sensitivity | Specificity |
|---|---|
| 100,0 | 3,20 |
| 95,0 | 16,00 |
| 90,0 | 30,40 |
| 80,0 | 54,40 |
| 60,5 | 80,0 |
| 50,8 | 90,0 |
| 42,2 | 95,0 |
| 4,3 | 100,0 |

### CE

**Area Under the Curve**

Test Result Variable(s): M48

| Area | Std. Error[a] | Asymptotic Sig.[b] | Asymptotic 95% Confidence Interval | |
|---|---|---|---|---|
| | | | Lower Bound | Upper Bound |
| ,721 | ,040 | ,000 | ,642 | ,800 |

a. Under the nonparametric assumption

b. Null hypothesis: true area = 0.5

| Sensitivity | Specificity |
|---|---|
| 100,0 | 0,00 |
| 95,0 | 14,81 |
| 90,0 | 23,46 |
| 80,0 | 50,62 |
| 53,1 | 80,0 |
| 39,5 | 90,0 |
| 30,9 | 95,0 |
| 3,7 | 100,0 |

### CL

**Area Under the Curve**

Test Result Variable(s): M48

| Area | Std. Error[a] | Asymptotic Sig.[b] | Asymptotic 95% Confidence Interval | |
|---|---|---|---|---|
| | | | Lower Bound | Upper Bound |
| ,897 | ,022 | ,000 | ,854 | ,941 |

a. Under the nonparametric assumption

b. Null hypothesis: true area = 0.5

| Sensitivity | Specificity |
|---|---|
| 100,0 | 10,38 |
| 95,0 | 52,83 |
| 90,0 | 75,47 |
| 80,0 | 85,85 |
| 87,5 | 80,0 |
| 69,2 | 90,0 |
| 54,8 | 95,0 |
| 10,6 | 100,0 |

**Figure 6**

A

ROC Curve

(Sensitivity vs 1 - Specificity plot)

B  BSI0221 (ACT), CYFRA

C  $\hat{P} = \left( \dfrac{1}{1 + e^{-(0.105 - 0.037 * BSI0221.2 + 0.289 * CYFRA)}} \right) * 100$

D

**Area Under the Curve**

Test Result Variable(s): Predicted probability (Total) - M13

| Area | Std. Error[a] | Asymptotic Sig.[b] | Asymptotic 95% Confidence Interval | |
|---|---|---|---|---|
| | | | Lower Bound | Upper Bound |
| ,845 | ,025 | ,000 | ,796 | ,894 |

a. Under the nonparametric assumption

b. Null hypothesis: true area = 0.5

| Cuttof | Sensitivity | Specificity |
|---|---|---|
| 0,068 | 100,0 | 3,7 |
| 0,130 | 95,0 | 31,2 |
| 0,147 | 90,0 | 38,5 |
| 0,283 | 80,0 | 79,4 |
| 0,295 | 78,6 | 80,0 |
| 0,392 | 65,0 | 90,0 |
| 0,457 | 54,4 | 95,0 |
| 1,000 | 3,9 | 100,0 |

E

**Case Processing Summary**

| Sample_type | Valid N (listwise) |
|---|---|
| Positive[a] | 103 |
| Negative | 218 |

F

Model prediction on BD data - M13

Sample type
- Control
- LC_Stage_I
- LC_Stage_II
- LC_Stage_IIIA
- LC_Stage_IIIB
- LC_Stage_IV

**Figure 7**

**Figure 8**

## A

ROC Curve

## D

**Area Under the Curve**

Test Result Variable(s): Predicted probability (Total) - M9

| Area | Std. Error[a] | Asymptotic Sig.[b] | Asymptotic 95% Confidence Interval | |
|---|---|---|---|---|
| | | | Lower Bound | Upper Bound |
| ,855 | ,027 | ,000 | ,803 | ,908 |

a. Under the nonparametric assumption

b. Null hypothesis: true area = 0.5

| Cuttof | Sensitivity | Specificity |
|---|---|---|
| 0,009 | 100,0 | 3,7 |
| 0,089 | 95,0 | 46,8 |
| 0,121 | 90,0 | 55,5 |
| 0,245 | 80,0 | 78,9 |
| 0,252 | 78,6 | 80,0 |
| 0,422 | 55,7 | 90,0 |
| 0,510 | 42,9 | 95,0 |
| 0,925 | 10,0 | 100,0 |

## B

BSI0144 (ACT), BSI0214 (ApoB100), BSI0221 (ACT), BSI0581 (C9), BSI0759(A2HSGP), BSI1186 (C4BP), CYFRA

## C

$$\hat{P} = \left(\frac{1}{1 + e^{-(1.721 - 0.002 \cdot BSI0144.4 - 0.032 \cdot BSI0214.1 + 0.059 \cdot BSI0221.2 - 0.036 \cdot BSI0581.2 + 0.010 \cdot BSI0759.1 - 0.062 \cdot BSI1186.1 + 0.156 \cdot CYFRA)}}\right) * 100$$

## E

**Case Processing Summary**

| Sample_type | Valid N (listwise) |
|---|---|
| Positive[a] | 70 |
| Negative | 218 |

## F

Model prediction data - LC6_M9

Sample type
- Control
- LC_Stage_I
- LC_Stage_II
- LC_Stage_III/A
- LC_Stage_III/B
- LC_Stage_IV

**Figure 9**

EP 3 899 541 B1

| Output | REFERENCE (Model Ext M63) | | QPLC Stat Model Ext M61 | | | | QPLC Stat Model M48 | | | |
|---|---|---|---|---|---|---|---|---|---|---|
| | Model_A | Model_B | Model_A | Difference (Model_A_Ext M63) | Model_B | Difference (Model_B_Ext M63) | Model_A | Difference (Model_A_Ext M63) | Model_B | Difference (Model_B_Ext M63) |
| specificity: | 85.9% | 95.7% | 85.7% | 99.7% | 95.7% | 99.9% | 88.5% | 103.0% | 96.5% | 100.8% |
| sensitivity: | 94.1% | 75.8% | 93.1% | 98.9% | 73.2% | 96.5% | 94.0% | 99.9% | 75.7% | 99.8% |
| Number of tests | 100000 | 100000 | 100000 | 100.0% | 100000 | 100.0% | 100000 | 100.0% | 100000 | 100.0% |
| Correctly identified LC (TP) | 1411 | 1138 | 1396 | 98.9% | 1097 | 96.5% | 1410 | 99.9% | 1135 | 99.8% |
| Missed LC (FN) | 89 | 362 | 104 | 117.2% | 403 | 111.1% | 90 | 101.1% | 365 | 100.7% |
| Correct negative (TN) | 84642 | 94302 | 84401 | 99.7% | 94231 | 99.9% | 87172 | 103.0% | 95054 | 100.8% |
| False positive (FP) | 13858 | 4198 | 14099 | 101.7% | 4269 | 101.7% | 11328 | 81.7% | 3446 | 82.1% |
| PPV | 9.24% | 21.32% | 9.01% | 97.5% | 20.45% | 95.9% | 11.07% | 119.8% | 24.78% | 116.2% |
| NPV | 99.90% | 99.62% | 99.88% | 100.0% | 99.57% | 100.0% | 99.90% | 100.0% | 99.62% | 100.0% |
| Number of LDCT/Chest X-ray | 170525 | 171960 | 172145 | 101.0% | 173581 | 100.9% | 154818 | 90.8% | 155963 | 90.7% |
| Number of QPLC tests. | 100000 | 100000 | 100000 | 100.0% | 100000 | 100.0% | 100000 | 100.0% | 100000 | 100.0% |
| Number of biopsy | 15269 | 5335 | 15495 | 101.5% | 5367 | 100.6% | 12738 | 83.4% | 4581 | 85.9% |

Reference Model_A (Ext M63) combined with 2 LDCT ("A"reference here)

Reference Model_B (Ext M63): 2 C X-ray "B" reference here
Model_A: LDCT + QPLC with 2 threshold+2nd LDCT
Model_B: C X-ray + QPLC with 2 threshold+2nd C X-ray

| EXT:63: | | |
|---|---|---|
| QM_BSI0144 | QP_BSI0782 | CYFRA |
| QM_BSI0581 | QP_BSI0116 | CRP |
| QM_BSI0186 | QP_BSI1186 | Albumin |
| QP_BSI0759 | QP_BSI0221 | |
| QP_BSI0581 | QP_BSI2487 | |
| QM_BSI0116 | | |

2 tracers

| ExM61: QM504 | |
|---|---|
| BSI0144 | CYFRA |
| BSI0581 | CRP |
| BSI0186 | Albumin |
| BSI2487 | |
| BSI0190 | |
| BSI0782 | |

1 (mixed) tracer

| M48- QP507 | |
|---|---|
| QP_BSI0581 | CYFRA |
| QP_BSI1186 | |
| QP_BSI0759 | |
| QP_BSI0782 | |

1 (depleted) tracer

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011015602 A
- EP 2772759 A
- US 8512959 B2, Takacs, L.
- EP 1802981 B1, Guttman, A. & Takacs, L.

### Non-patent literature cited in the description

- **KARU, A. E ; BELL, C. W. ; CHIN, T. E.** Recombinant Antibody Technology. *ILAR J.,* 1995, vol. 37, 132-141
- **CANDIA, J. et al.** Assessment of Variability in the SOMAscan Assay. *Sci. Rep.,* 2017, vol. 7, 14248
- **UHLÉN, M. et al.** A Human Protein Atlas for Normal and Cancer Tissues Based on Antibody Proteomics. *Mol. Cell. Proteomics,* 2005, vol. 4, 1920-1932
- **WANG, D. et al.** Antigen identification and characterization of lung cancer specific monoclonal antibodies produced by mAb proteomics. *J. Proteome Res.,* 2010, vol. 9, 1834-1842
- **STUDER, R. A. ; DESSAILLY, B. H. ; ORENGO, C. A.** Residue mutations and their impact on protein structure and function: detecting beneficial and pathogenic changes. *Biochem. J.,* 2013, vol. 449, 581-594
- **GUERGOVA-KURAS, M. et al.** Discovery of lung cancer biomarkers by profiling the plasma proteome with monoclonal antibody libraries. *Mol. {&} Cell. proteomics MCP,* 2011, vol. 10, M111.010298
- **HAJDÚ, I. et al.** Monoclonal antibody proteomics: Use of antibody mimotope displaying phages and the relevant synthetic peptides for mAb scouting. *Immunol. Lett,* 2014, vol. 160, 172-177
- **VAN KLAVEREN, R. J. et al.** Management of Lung Nodules Detected by Volume CT Scanning. *N. Engl. J. Med.,* 2009, vol. 361, 2221-2229
- **PEDERSEN, J. H. et al.** The Danish Randomized Lung Cancer CT Screening Trial-Overall Design and Results of the Prevalence Round. *J. Thorac. Oncol.,* 2009, vol. 4, 608-614
- **HOREWEG, N ; NACKAERTS, K ; OUDKERK, M. ; DE KONING, H. J.** Low-dose computed tomography screening for lung cancer: results of the first screening round. *J. Comp. Eff. Res.,* 2013, vol. 2, 433-436
- **FITZGERALD, S. P. ; LAMONT, J. V ; MCCONNELL, R. I ; BENCHIKH, E. O.** Development of a high-throughput automated analyzer using biochip array technology. *Clin. Chem.,* 2005, vol. 51, 1165-76